# EUROPEAN PATENT APPLICATION

(11) **EP 4 122 478 A1**
(43) Date of publication of application: **25.01.2023**
(21) Application number: 22180454.5
(22) Date of filing: 18.02.2017
(51) Int. Cl.: A61K 35/76, A61K 39/00, A61K 39/12, A61K 35/763, A61P 35/00

(54) **COMPOSITIONS AND METHODS OF USING STAT1/3 INHIBITORS WITH ONCOLYTIC HERPES VIRUS**

(30) Priority: 19.02.2016 US 201662297739 P; 26.09.2016 US 201662399989 P
(62) Divisional of application: 17754008.5
(71) Applicant: Virogin Biotech Canada Ltd, Richmond, BC V6V 2J8 (CA)
(72) Inventor: JIA, William, Burnaby, V5B 3J9 (CA); DELWAR, Zahid, Richmond, V6Y 1Y7 (CA)
(74) Representative: Forresters IP LLP

(57) **Abstract**

The present disclosure relates to the use of an oncolytic virus, such as HSV, and a STAT1/3 inhibitor, such as nifuroxazide and C16, in the treatment of cancer. Therapeutic compositions are provided that may be used to prevent, treat, or ameliorate the effects of a targeted cancer. Methods of using such compositions are also disclosed, such as methods of using the therapeutic compositions for improving efficacy of an oncolytic virotherapy (and for preventing macrophage and microglia inhibition of oncolytic viral activity).

## Description

### CROSS-REFERENCE TO RELATED APPLICATIONS

This patent application claims the benefit under 35 U.S.C. § 119(e) of U.S. Provisional Patent Application No. 62/297,739 filed February 19, 2016, and U.S. Provisional Patent Application No. 62/399,989 filed September 26, 2016, both of which are incorporated in their entirety.

### FIELD OF THE INVENTION

This patent application relates generally to compositions and to treatment of cancer with oncolytic HSV and a STAT1/3 inhibitor.

### BACKGROUND

Oncolytic viruses (OVs) have been a therapeutic arsenal to specifically destroy cancer cells through oncolysis, which is a killing mechanism characterized by cancer cell lysis through the course of virus lytic replication. In addition to the direct cell killing by the virus, it has been demonstrated that virally induced immune response plays a pivotal role in OV therapy. As OVs can kill cancer cells via a mechanism distinct from the killing effects of conventional chemotherapy and radiotherapy, OVs are potentially ideal to treat cancers that are non-responsive to conventional treatment. Among the various OVs, herpes simplex virus type 1 based OVs are the farthest advanced, e.g., a herpes virus-based OV (T-Vec) has been approved by the U.S. FDA. for the treatment of melanoma.

The most frequently investigated oncolytic virus to treat cancer, such as glioblastoma multiforme (GBM), is a mutant HSV-1 (herpes simplex virus type 1) with a deletion γ34.5. In spite of an excellent safety profile, clinical efficacy of many oHSV-1s has been disappointing.

The present invention overcomes shortcomings of current commercial oncolytic viruses, and further provides additional unexpected benefits.

### SUMMARY

Briefly stated, the present invention provides compositions and methods of treating cancer. In certain embodiments, methods are provided comprising the simultaneous or sequential administration of an oncolytic virus and a STAT1/3 inhibitor. In embodiments, the cancer is a breast cancer, brain cancer (e.g., glioblastoma), colon cancer, lung cancer, or prostate cancer. In embodiments, the oncolytic virus is herpes simplex virus, and in certain embodiments, the HSV is HSV-1. Within certain embodiments of the invention, the oncolytic virus is a HSV-1 with a defective viral ribonuclease reductase gene, and optionally, an otherwise intact ICP34.5 gene.

In embodiments all copies of the ICP34.5 gene in the genome of the oncolytic herpes simplex virus are modified such that the ICP34.5 gene is incapable of expressing a functional ICP34.5 gene product. In other embodiments, the ICP6 gene is modified such that the ICP6 gene is incapable of expressing a functional ICP6 gene product. In other embodiments, the ICP47 gene is modified such that the ICP47 gene is incapable of expressing a functional ICP47 gene product. In some embodiments, the oHSV has modifications of both the ICP34.5 and ICP47 genes. In embodiments, the oncolytic herpes simplex virus is a mutant of strain 17. In yet other embodiments the oncolytic virus is HSV-1 strain HrR3. In embodiments, the STAT1/3 inhibitor is a nitrofuran; in certain embodiments, the nitrofuran is nifuroxazides or a derivative or analog thereof. In other embodiments, the STAT1/3 inhibitor is C16 or a derivative or analog thereof.

Claims are also directed to pharmaceutical compositions comprising an oncolytic virus and a STAT1/3 inhibitor. In embodiments, the oncolytic virus is herpes simplex virus, and in certain embodiments, the HSV is HSV-1. Within certain embodiments of the invention, the oncolytic virus is a HSV-1 with a defective viral ribonuclease reductase gene, and optionally, an otherwise intact ICP34.5 gene. In embodiments all copies of the ICP34.5 gene in the genome of the oncolytic herpes simplex virus are modified such that the ICP34.5 gene is incapable of expressing a functional ICP34.5 gene product. In other embodiments, the ICP6 gene is modified such that the ICP6 gene is incapable of expressing a functional ICP6 gene product. In other embodiments, the ICP47 gene is modified such that the ICP47 gene is incapable of expressing a functional ICP47gene product. In some embodiments, the oHSV has modifications of both the ICP34.5 and ICP47 genes. In embodiments, the oncolytic herpes simplex virus is a mutant of strain 17. In embodiments, the STAT1/3 inhibitor is a nitrofuran; in certain embodiments, the nitrofuran is nifuroxazides or a derivative or analog thereof. In other embodiments, the STAT1/3 inhibitor is C16 or a derivative or analog thereof.

Claims are also directed to a kit comprising a predetermined amount of an oncolytic virus, which may be herpes simplex virus, and a predetermined amount of a therapeutic agent, wherein the therapeutic agent is STAT1/3 inhibitor. In embodiments, the oncolytic virus is herpes simplex virus, and in certain embodiments, the HSV is HSV-1. Within certain embodiments of the invention, the oncolytic virus is a HSV-1 with a defective viral ribonuclease reductase gene, and optionally an otherwise intact ICP34.5 gene. In other embodiments all copies of the ICP34.5 gene in the genome of the oncolytic herpes simplex virus are modified such that the ICP34.5 gene is incapable of expressing a functional ICP34.5 gene product. In other embodiments, the ICP47 gene is modified such that the ICP47 gene is incapable of expressing a functional ICP47gene product. In other embodiments, the ICP6 gene is modified such that the ICP6 gene is incapable of expressing a functional ICP6 gene product. In some embodiments, the oHSV has modifications of both the ICP34.5 and ICP47 genes. In embodiments, the oncolytic herpes simplex virus is a mutant of strain 17. In embodiments, the STAT1/3 inhibitor is a nitrofuran; in certain embodiments, the nitrofuran is nifuroxazides or a derivative or analog thereof. In other embodiments, the STAT1/3 inhibitor is C16 or a derivative or analog thereof.

The disclosure provides that the combination of an oncolytic virus and a STAT 1/3 phosphorylation inhibitor is particularly efficacious in preventing, treating, and/or ameliorating the effects of a cancer (e.g., a breast cancer, brain cancer (e.g., glioblastoma), colon cancer, lung cancer, or prostate cancer). In one aspect, a method is provided for improving efficacy of an oncolytic virotherapy, comprising the steps of (a) administering an oncolytic virus to a subject; and (b) administering a STAT1/3 inhibitor in an amount that is effective to reduce microglia- or macrophage-mediated suppression of replication of the oncolytic virus. In some embodiments, the oncolytic virus and STAT1/3 inhibitor are administered together; in others, the oncolytic virus and STAT1/3 inhibitor are administered in series. In certain embodiments, the STAT1/3 inhibitor is C16, salt forms thereof, prodrugs thereof, or derivatives thereof. In other embodiments, the STAT1/3 inhibitor is nitrofuran, and in specific embodiments, the nitrofuran is nifuroxazide or a derivative or analog thereof.

The co-administration of a STAT 1/3 phosphorylation inhibitor (such as C16 and nifuroxazide), along with an oncolytic virus, is effective to prevent macrophage and microglia inhibition of oncolytic viral activity, thereby enhancing the efficacy of the oncolytic virus.

In addition to the compositions described herein, various methods of using such compositions are provided For examples, a method for improving efficacy of an oncolytic virotherapy, and a method for improving oncolytic activity in a cancer cell (and for preventing macrophage and microglia inhibition of oncolytic viral activity in a cancer cell) are provided.

These and other aspects of the present invention will become evident upon reference to the following detailed description and attached drawings.

This Brief Summary has been provided to introduce certain concepts in a simplified form that are further described in detail below in the Detailed Description. Except where otherwise expressly stated, this Brief Summary is not intended to identify key or essential features of the claimed subject matter, nor is it intended to limit the scope of the claimed subject matter.

The details of one or more embodiments are set forth in the description below. The features illustrated or described in connection with one exemplary embodiment may be combined with the features of other embodiments. Thus, any of the various embodiments described herein can be combined to provide further embodiments. Aspects of the embodiments can be modified, if necessary to employ concepts of the various patents, applications and publications as identified herein to provide yet further embodiments. Other features, objects and advantages will be apparent from the description, the drawings, and the claims.

### BRIEF DESCRIPTION OF THE SEQUENCE LISTING

SEQ ID NO:1 Forward PCR primer for ICP4.
SEQ ID NO:2 Reverse PCR primer for ICP4.
SEQ ID NO:3 Forward PCR primer for ICP27.
SEQ ID NO:4 Reverse PCR primer for ICP27.
SEQ ID NO:5 Forward PCR primer for β-actin.
SEQ ID NO:6 Reverse PCR primer for β-actin.
SEQ ID NO:7 Forward PCR primer for ICP8.
SEQ ID NO:8 Reverse PCR primer for ICP8.
SEQ ID NO:9 Forward PCR primer for GC.
SEQ ID NO:10 Reverse PCR primer for GC.
SEQ ID NO:11 Forward PCR primer for VP5.
SEQ ID NO:12 Reverse PCR primer for VP5.
SEQ ID NO:13 Forward PCR primer for ICP27.
SEQ ID NO:14 Reverse PCR primer for ICP27.
SEQ ID NO:15 Forward PCR primer for β-actin.
SEQ ID NO:16 Reverse PCR primer for β-actin.

### BRIEF DESCRIPTION OF THE DRAWINGS

Exemplary features of the present disclosure, its nature and various advantages will be apparent from the accompanying drawings and the following detailed description of various embodiments. Non-limiting and non-exhaustive embodiments are described with reference to the accompanying drawings, wherein like labels or reference numbers refer to like parts throughout the various views unless otherwise specified. The sizes and relative positions of elements in the drawings are not necessarily drawn to scale. For example, the shapes of various elements are selected, enlarged, and positioned to improve drawing legibility. The particular shapes of the elements as drawn have been selected for ease of recognition in the drawings. One or more embodiments are described hereinafter with reference to the accompanying drawings in which:

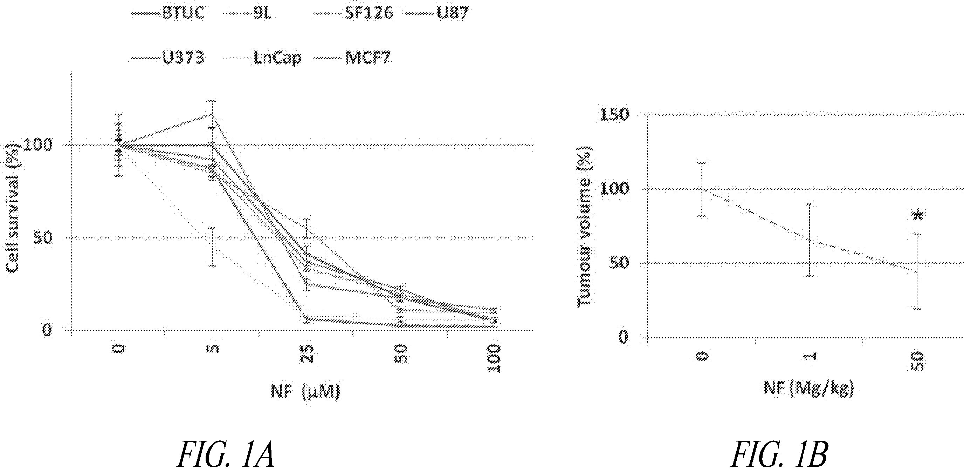

Figures 1A-1B are graphs that show an anti-tumor effect of nifuroxazide. (Fig. 1A) Cells were treated with indicated concentration of nifuroxazide for 72 hours. Cytotoxicity was measured by using MTT assay. (Fig. 1B) Subcutaneously U87 tumor bearing mice were peritoneally injected with indicated doses of nifuroxazide. Tumor size was measured using calipers at day 7 post nifuroxazide treatment.

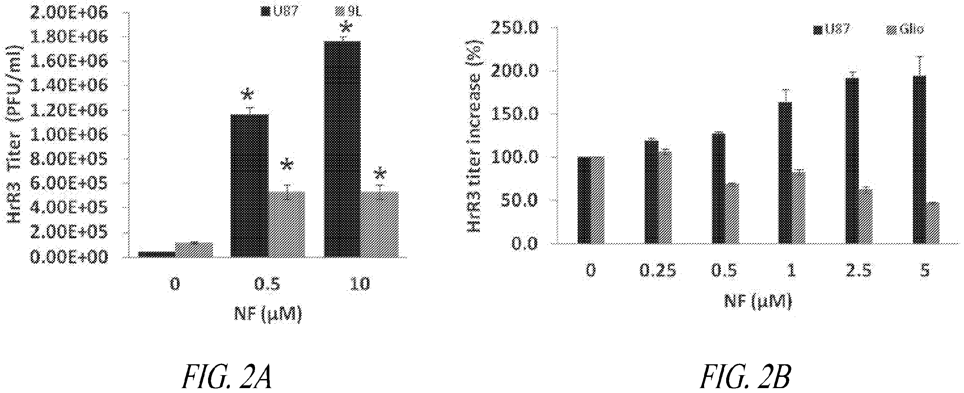

Figures 2A-2B contain charts that show the effect of nifuroxazide on replication of oHSV-1. (Fig. 2A) Indicated cells were infected with HrR3 viruses at MOI- 0.1. Viruses were harvested at 48 hours post-infected and titrated in vero cells. Data are presented as mean SD, * P<0.05 vs corresponding vehicle treatment. (Fig. 2B) U87 or glio cells were treated with HrR3 at a MOI of 1 and indicated concentration of nifuroxazide for 48 hours. Total DNA was extracted and subjected to qPCR to detect HSV-1 using ICP27 primer. nifuroxazide mediated oHSV-1 % increase was represented in the figure.

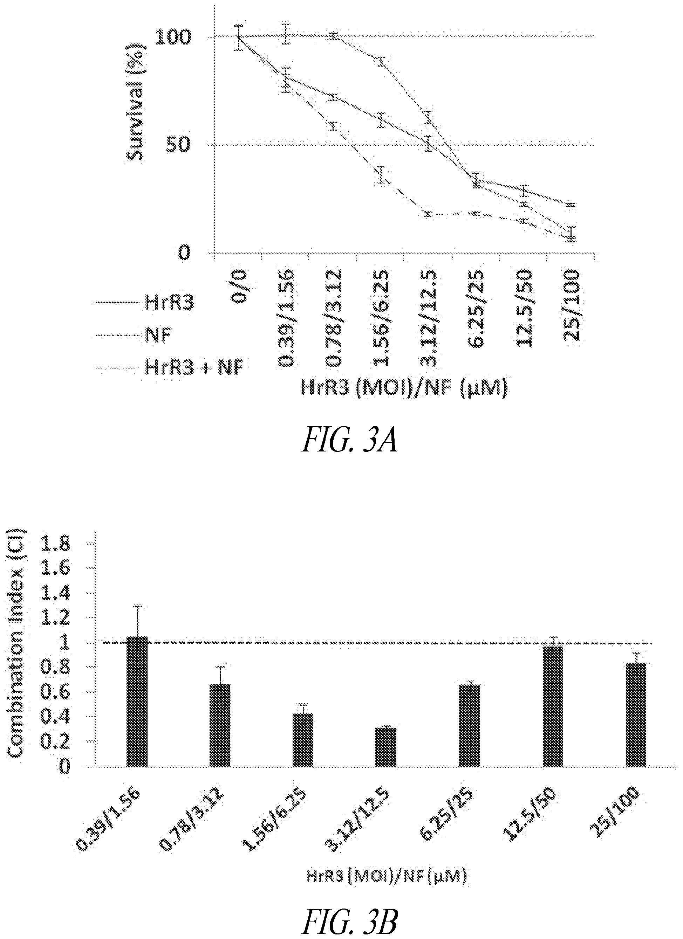

Figures 3A-3B are charts that show effect of nifuroxazide and oHSV on tumor cell proliferation. U87 cells were treated with indicated HrR3 and nifuroxazide doses. (Fig. 3A) Cytotoxicity was measured by MTT assay at 72 hours post-treatment. (Fig. 3B) Combination index (CI) was determined by using calcusyn software.

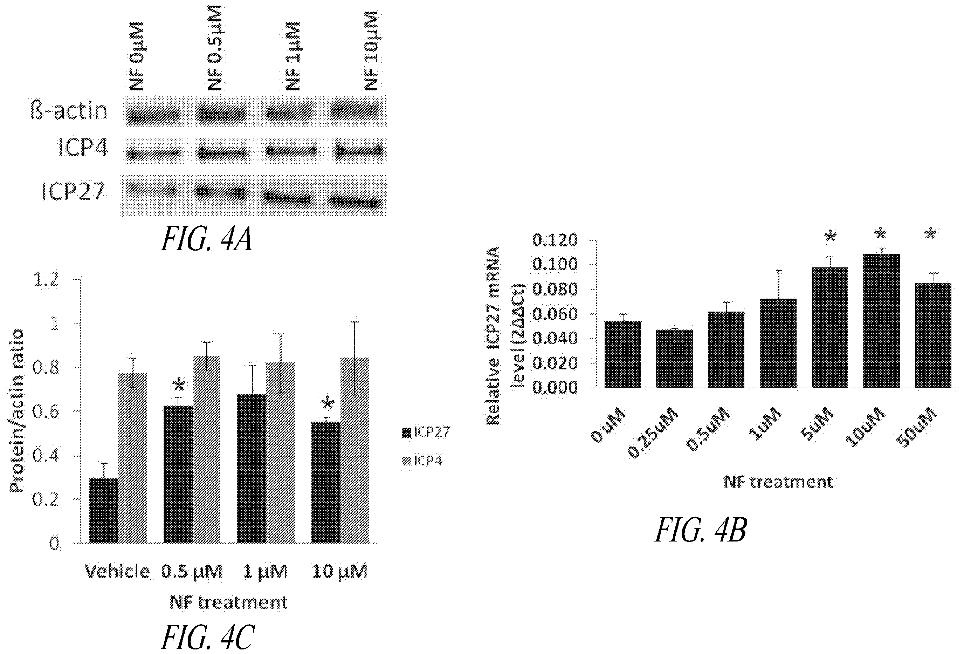

Figures 4A-4C show the effect of nifuroxazide on HSV-1 gene expression. (Fig. 4A) U87 cells were treated with HrR3 viruses at a MOI-3 and indicated doses of nifuroxazide. 24 hours post infected, total protein was extracted and subjected to western blot analysis. (Fig.4B) U87 cells were treated with HrR3 at a MOI-5 and indicated concentration of nifuroxazide for 6 hours. ICP27 mRNA level was detected by RT-qPCR. (Fig.4C) Relative mRNA level (2^{-ΔΔCT}) is shown. Data are presented as mean SD, * P<0.05 vs corresponding vehicle treatment.

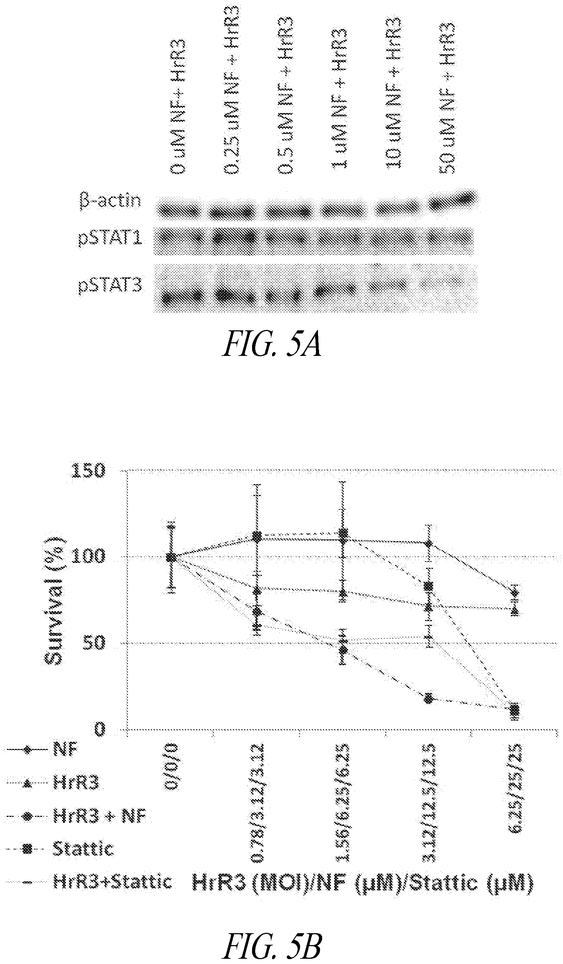

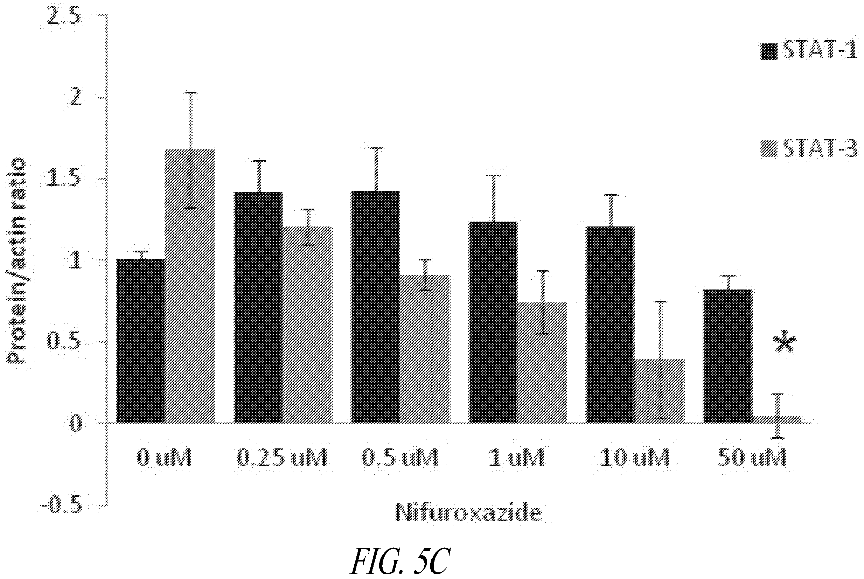

Figures 5A-5C depict the effect of nifuroxazide on STATs activation in oHSV-1 infected tumor cells. (Fig. 5A) U87 cells were treated with HrR3 at a MOI of 5 and indicated concentration of nifuroxazide for 6 hours. Extracted protein was then subjected to western blot assay. (Fig. 5B) U87 cells were treated with indicated concentration of HrR3 or nifuroxazide or stattic or in combination for 72 hours. Cytotoxicity was measured by MTT assay. (Fig. 5C) Relative mRNA level (2^{-ΔΔCT}) is shown. Data are presented as mean SD, * P<0.05 vs corresponding vehicle treatment.

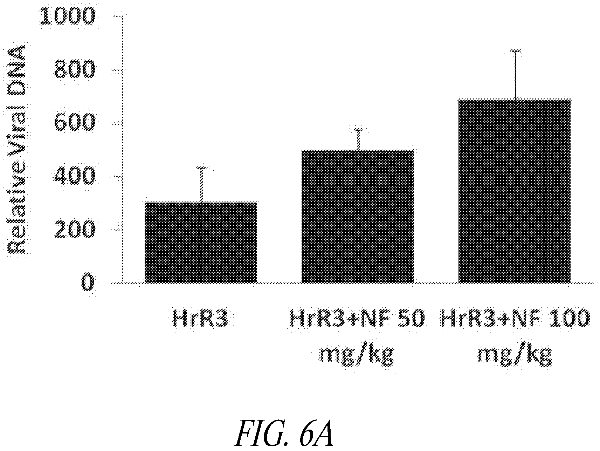

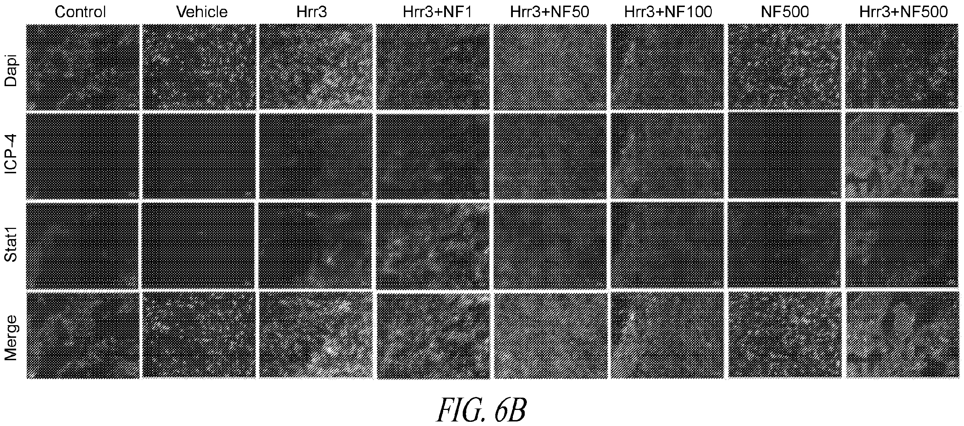

Figures 6A and 6B show results of in vivo dose dependent HrR3 replication augmentation by nifuroxazide. Subcutaneously U87 tumor bearing mice were intratumorally injected with single dose of HrR3 virus (n=2; 1X10^6 PFU/ml) or peritoneally injected indicated concentration of nifuroxazide or both combination. Tumors were harvested at day 10 post treatment (Fig. 6A) Total genomic DNA was extracted from the harvested tumors. Viral DNA particles (ICP27) were detected by qPCR and normalized to β-actin. (Fig. 6B) ICP4 and STAT1 expression were determined by immunohistochemistry assay on harvested tumor tissues. Stained sections were then imaged using confocal microscopy.

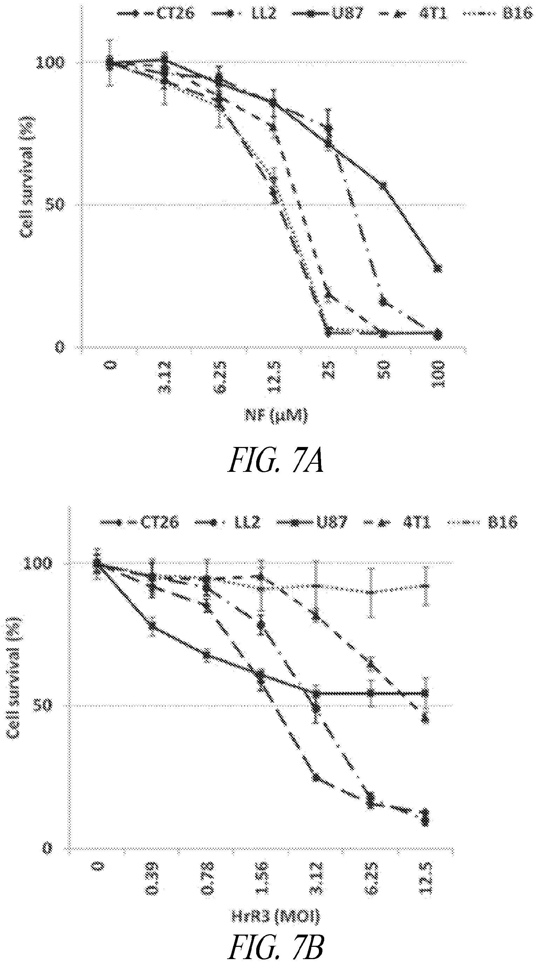

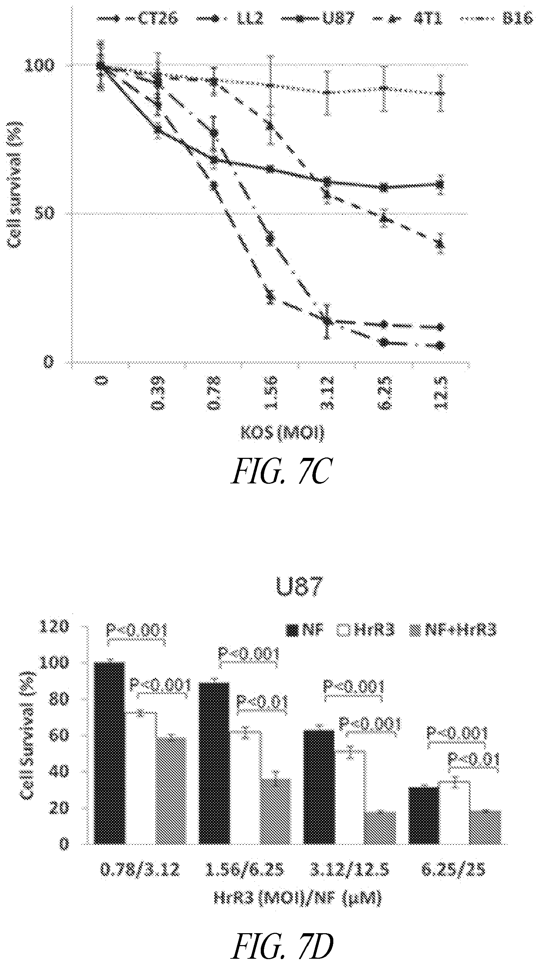

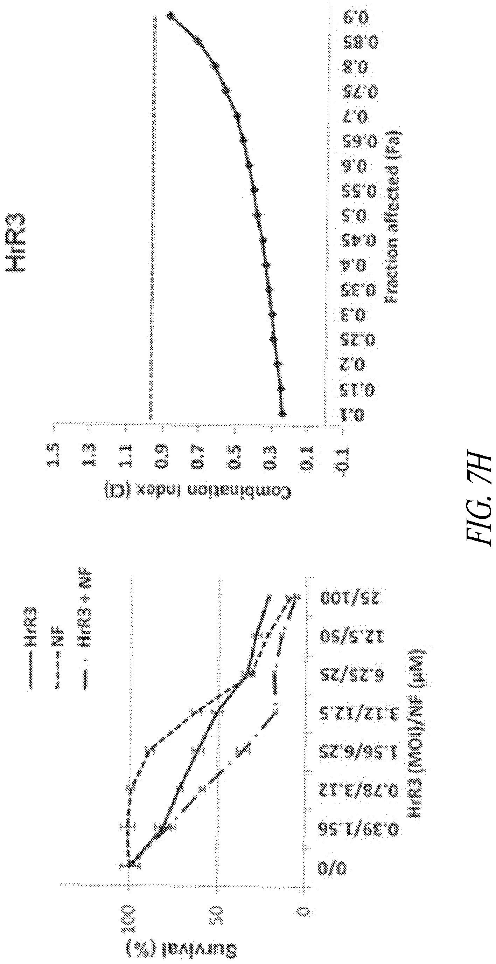

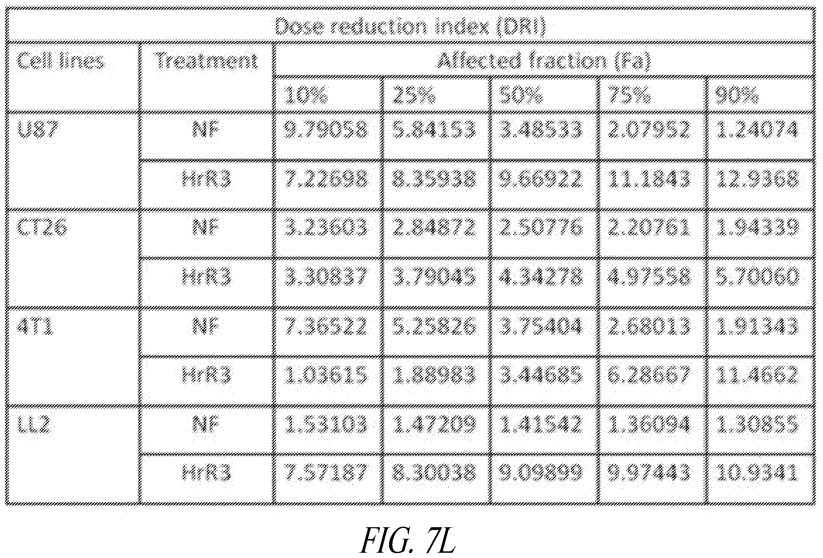

Figures 7A-7L show results of treatment of tumor cells with nifuroxazide and oHSV-1. Fig. 7A-7C show cell survival for tumor cells CT26, LL2 and U87 treated with nifuroxazide or HrR3 or KOS. Fig. 7D-7G show cell survival of tumor cells, U87, CT26, 4T1 and LL2, treated first with nifuroxazide and then HrR3 virus. Fig. 7H-7K show combination index (CI) values for tumor cells treated with varying doses of nifuroxazide or the viruses, HrR3 or KOS, or VG12TR, alone or in combination. Fig. 7L is a table presenting the dose reduction index for a combination of nifuroxazide and HrR3 virus.

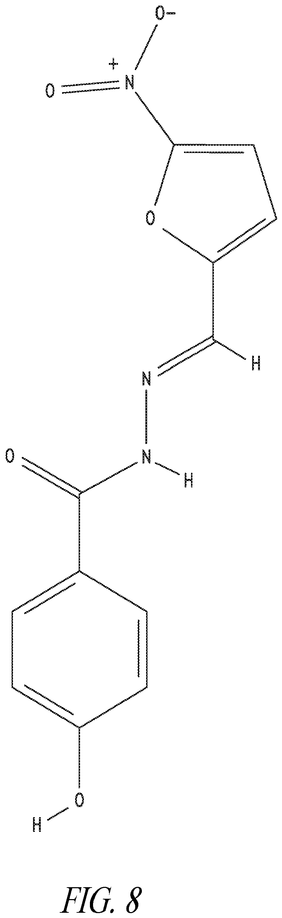

Figure 8 depicts the chemical structure of nifuroxazide.

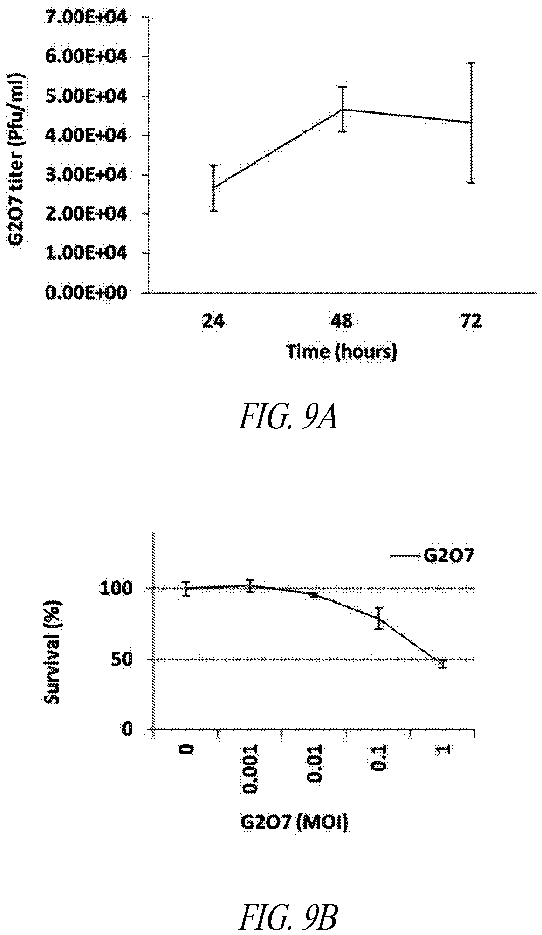

Figure 9A is a line graph showing G207 growth in U87 cells, as determined by a single step growth assay.
Figure 9B is a line graph showing the cytotoxic effect of G207, as measured by the MTT cell proliferation assay described herein at 3 days post-infection in U87 cells.

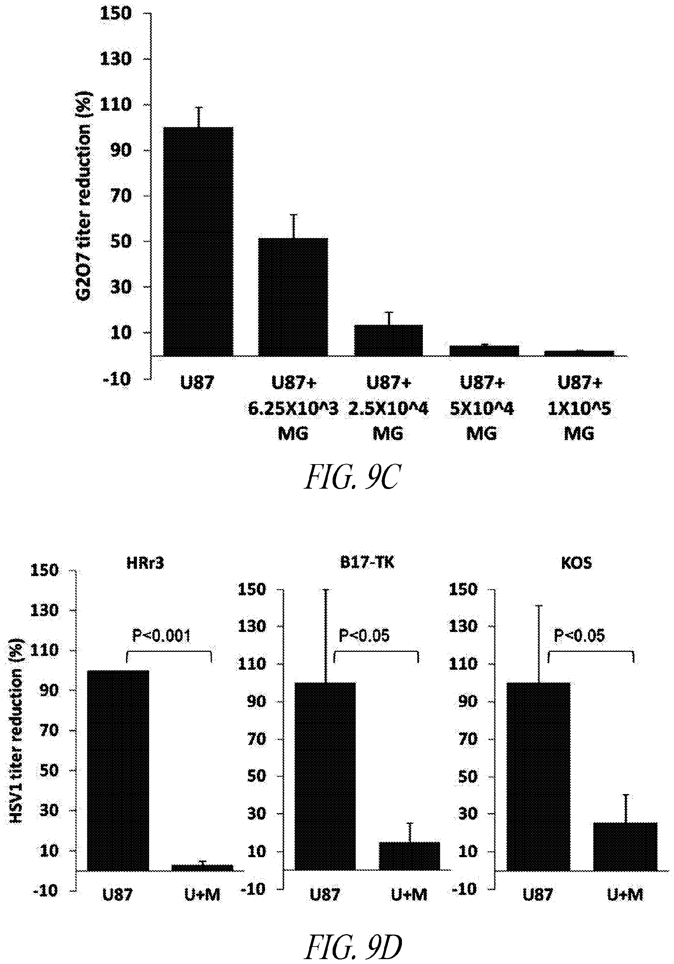

Figure 9C is a bar graph showing the effect of microglia on G207 growth in U87 (5X104) cells alone or with different numbers of rat primary microglia (MG) cells (the cells were infected with G207 at a multiplicity of infection (MOI) of 1).
Figure 9D is a bar graph showing the results of U87 (5X104) cells alone or U87 (5X104) + MG (5X104) co-culture being infected at an MOI=1 with ICP6 gene mutated oHSV-1 hRr3, TK deleted oHSV-1 b17-TK, or wild type HSV-1 KOS (virus replication efficiency was determined by single step virus growth assay at 4 days post-infection (means ± SD)).

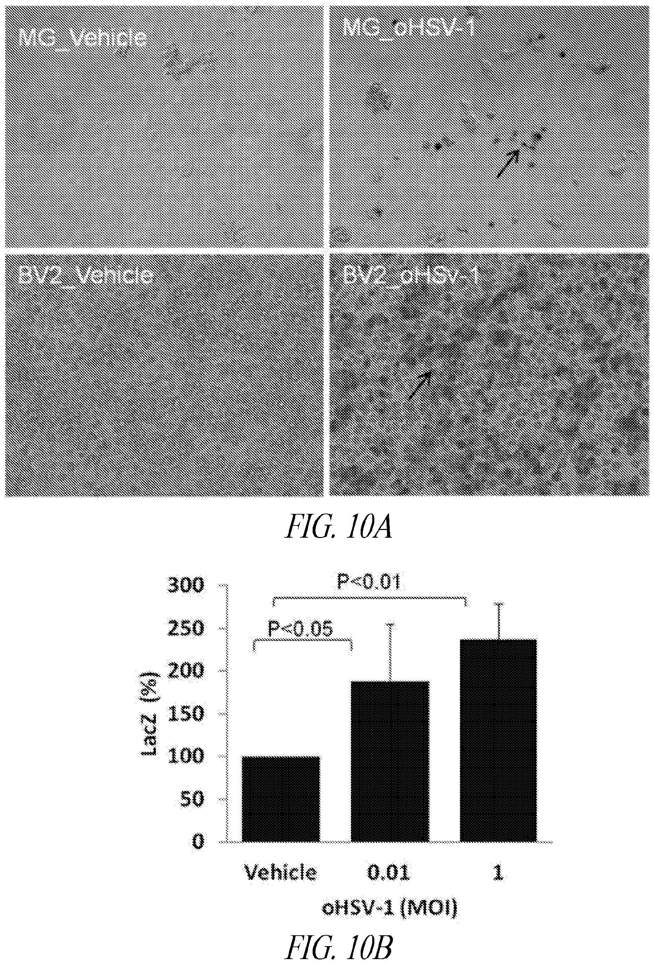

Figure 10A shows histology results of rat primary microglia (MG) or BV2 cells being infected with G207 virus at an MOI of 3 or mock preparations (to detect viral reporter gene expression, cells were stained for β-galactosidase (arrow) after 24 hours of infection).
Figure 10B is a bar graph showing the results of BV2 cells that were infected with a mock or G207 at indicated MOIs for 24 hours, with LacZ expression then being measured at 420 nm.

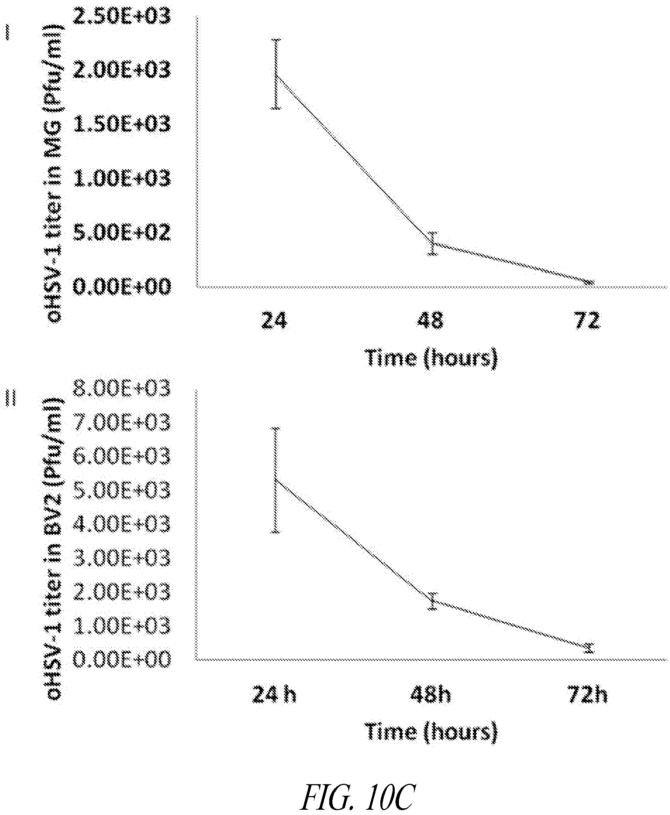

Figure 10C shows line graphs of the results of MG or BV2 microglia cells that were infected with G207 at an MOI of 1 to examine the replication capacity of G207. Viruses were harvested at 24 hours, 48 hours, and 72 hours post-infection and then titrated on Vero cells.

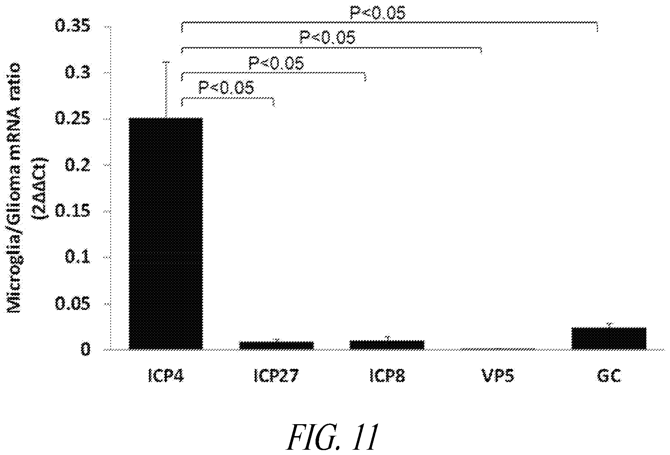

Figure 11 is a bar graph showing oHSV-1 gene expression in microglia cells. mRNA levels of different genes of G207 virus (ICP4, ICP27, ICP8, VP5 and GC) were detected by RT-qPCR. Ratios of relative mRNA level (2-ΔΔCT) of G207 infected BV2 and U87 cells (BV2/U87) are presented in the bar graphs.

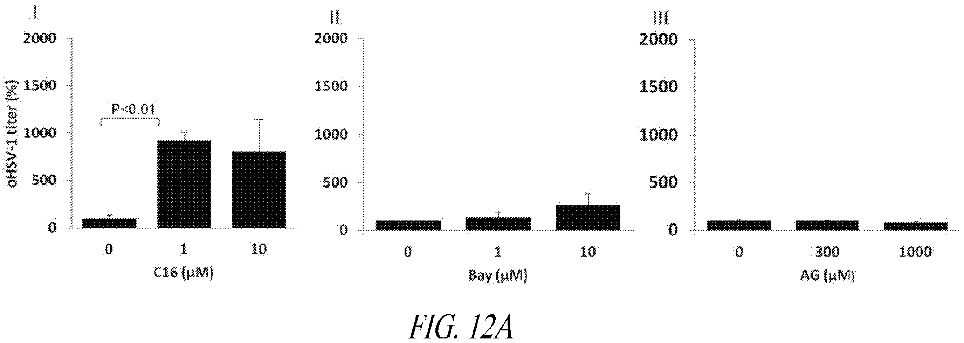

Figure 12A contains bar graphs showing the results of BV2 cells that were pre-treated with indicated chemical compounds for 1-2 hours and then infected with oHSV-1 at an MOI of 1. Viruses were harvested after 48 hours of oHSV-1 (G207) and indicated chemical inhibitor treatment.

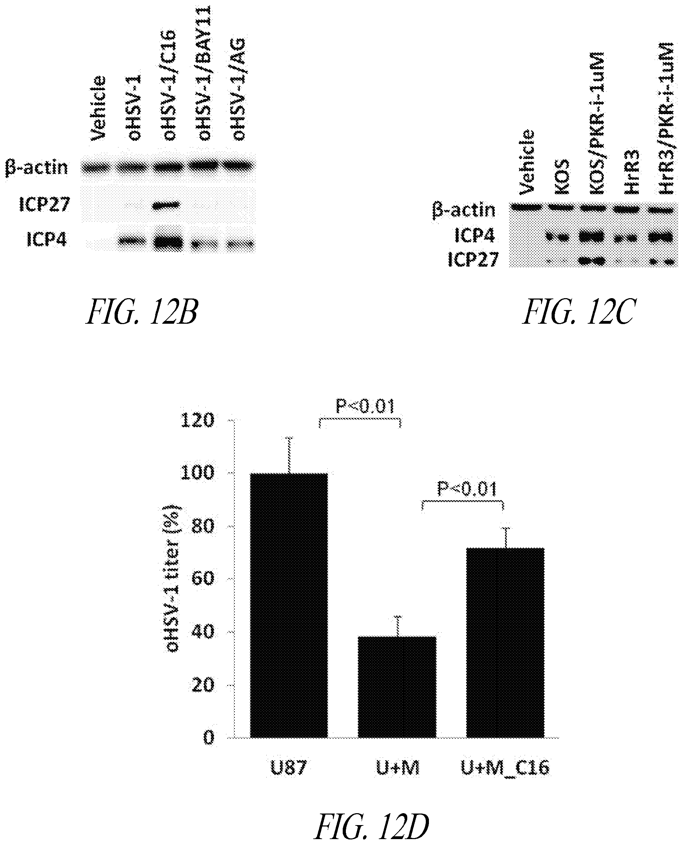

Figure 12B depicts an electrophoresis gel showing total proteins that were harvested from G2O7 infected microglia cells at 24 hours post treatment with either 1 µM of C16, 1 µM of Bay11, or 100 µM of AG. ICP27, ICP4, and β-actin expression was measured by western blot assay. U87 and MG co-culture were pretreated with 0.5 µM of C16 for 1 hour.
Figure 12C depicts an electrophoresis gel showing total proteins that were harvested from HrR3 or KOS infected microglia cells at 24 hours post-treatment with 1µM of PKR-i (C16). ICP27, ICP4, and β-actin expression was measured by western blot assay. U87 and MG co-culture were pretreated with 0.5 µM of C16 for 1 hour.
Figure 12D is a bar graph showing G207 viral replication increasing by 33% in glioma-microglia co-cultures treated with C16.

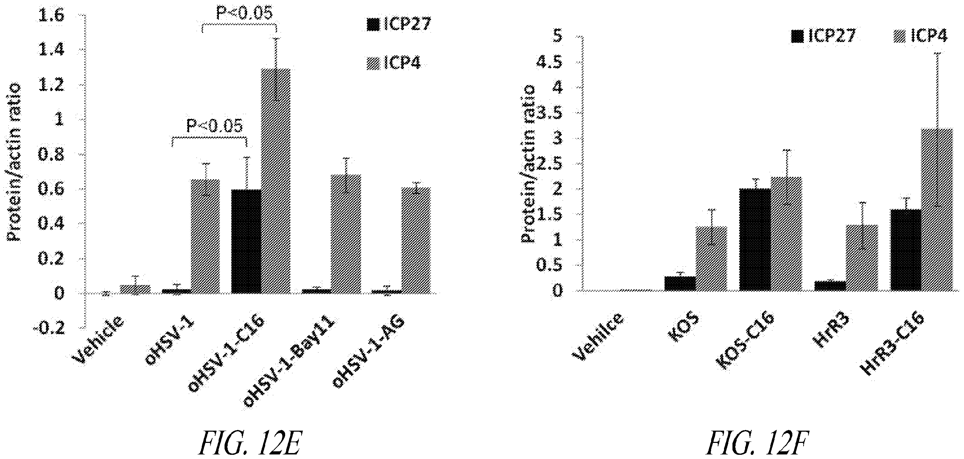

Figure 12E is a bar graph showing the results of BV2 cells that were pre-treated with indicated chemical compounds for 1-2 hours and then infected with HrR3 or KOS at an MOI of 1. Viruses were harvested after 48 hours of HrR3 / KOS and indicated chemical inhibitor treatment. After 48 hours of treatment with HrR3 / KOS and C16, viruses were harvested and titrated by plaque forming assay on vero cells.
Figure 12F is a bar graph showing the results of BV2 cells that were pre-treated with indicated chemical compounds for 1-2 hours and then infected with oHSV-1 at an MOI of 1. Viruses were harvested after 48 hours of oHSV-1 (G207) and indicated chemical inhibitor treatment. After 48 hours of treatment with oHSV-1 (G207) and C16, viruses were harvested and titrated by plaque forming assay on vero cells.

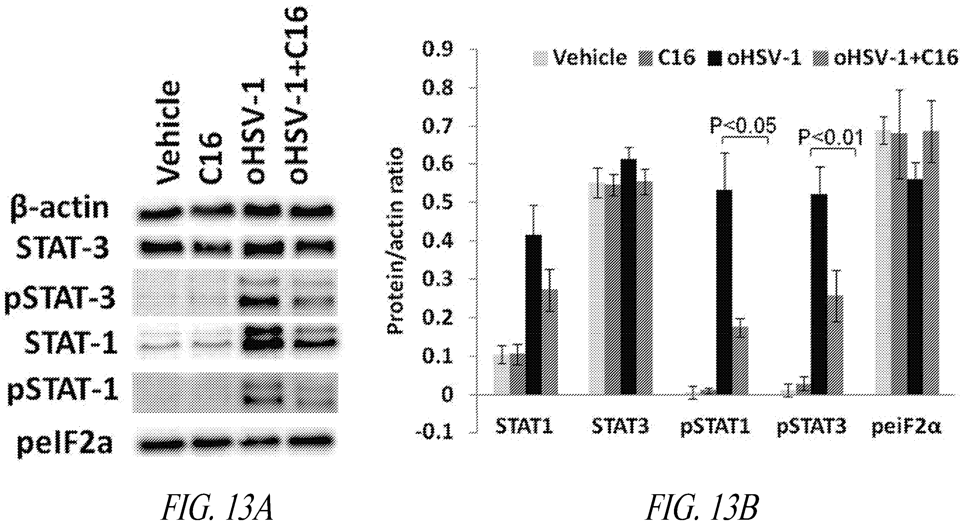

Figure 13A depicts an electrophoresis gel that shows C16 inhibits STAT 1 and STAT 3 phosphorylation. BV2 cells were pre-treated with vehicle or C16 (1µM) for 2 hours and then incubated with vehicle or oHSV-1 (G2O7) at an MOI of 1 or oHSV-1 and C16 combination for 24 hours. Total protein was harvested and subjected to western blot assay. β-actin expression was used for loading control (means ± SE).
Figure 13B is a bar graph that summarizes the results of Figure 5A.

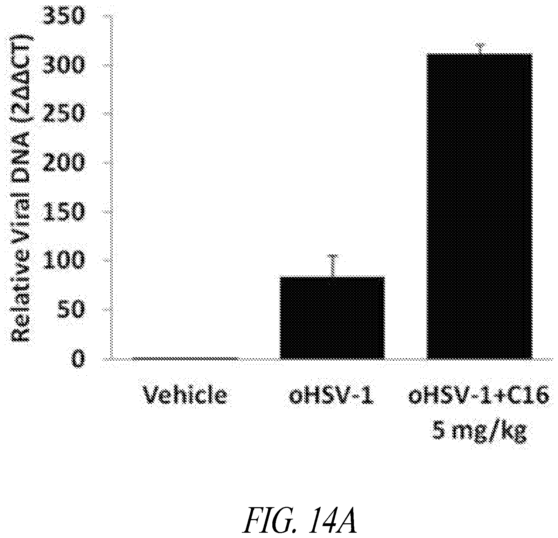

Figure 14A is a bar graph showing that C16 triggers oHSV-1 protein expression in microglia / macrophages and enhances virus concentration in glioblastoma in vivo. U87 xenograft mice were treated with vehicle or 5 mg/kg C16 with or without 6X106 PFU/ml oHSV-1 (G207) viruses (n=2). Two doses of G207 or vehicle and 4 doses (in every 3 or 4 days) of C16 was administered in total. Tumors were harvested at day 13 post-treatment. Total genomic DNA was extracted from the harvested tumors (n=2) and viral DNA (ICP27) was measured by qPCR (normalized to β-actin). Data shown are relative 2-ΔΔCT value ± SE.

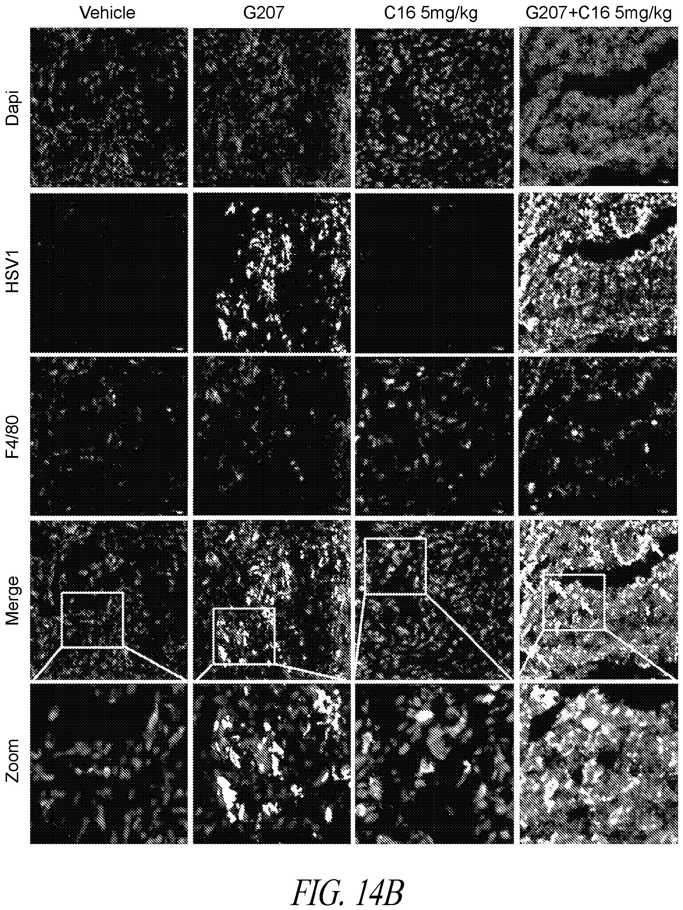

Figure 14B shows immunostaining of infiltrated macrophages (f4/80) and replicable HSV-1s in U87 tumors of mice.

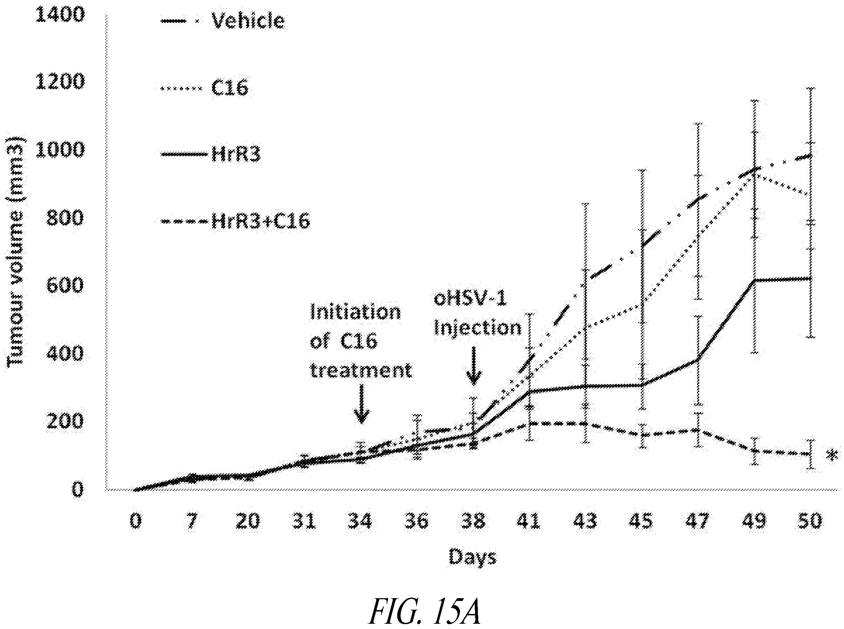

Figure 15A: A line graph showing tumor volume of U87 xenograft mice that were treated with vehicle or 5 mg/kg C16 with or without a single dose of 1X108 PFU/ml oHSV-1 (HrR3) viruses (n=5). Multiple doses of vehicle or C16 were injected intraperitoneally (IP) every other day. The (*) designation represents significant (P≤ 0.05) tumor regression by oHSV-1 and C16 combination versus vehicle or individual C16 or individual oHSV-1 treatment (± SE).

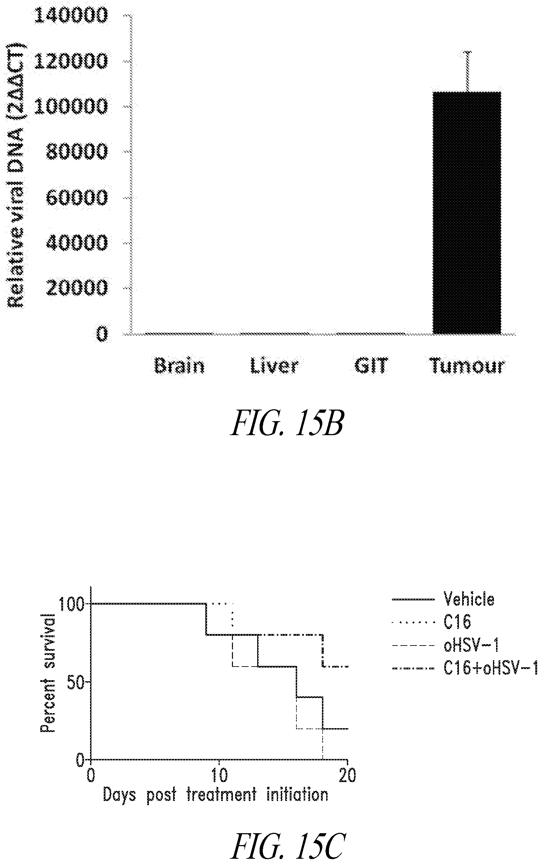

Figure 15B is a bar graph showing total genomic DNA that was extracted from the harvested organs (n=3). Viral DNA (ICP27) was measured by qPCR (normalized to β-actin). Data shown are relative 2-ΔΔCT value ± SE.
Figure 15C is a line graph showing a Kaplan-meier survival analysis of the mice - subjects of the Examples summarized in Figures 7A and 7B.

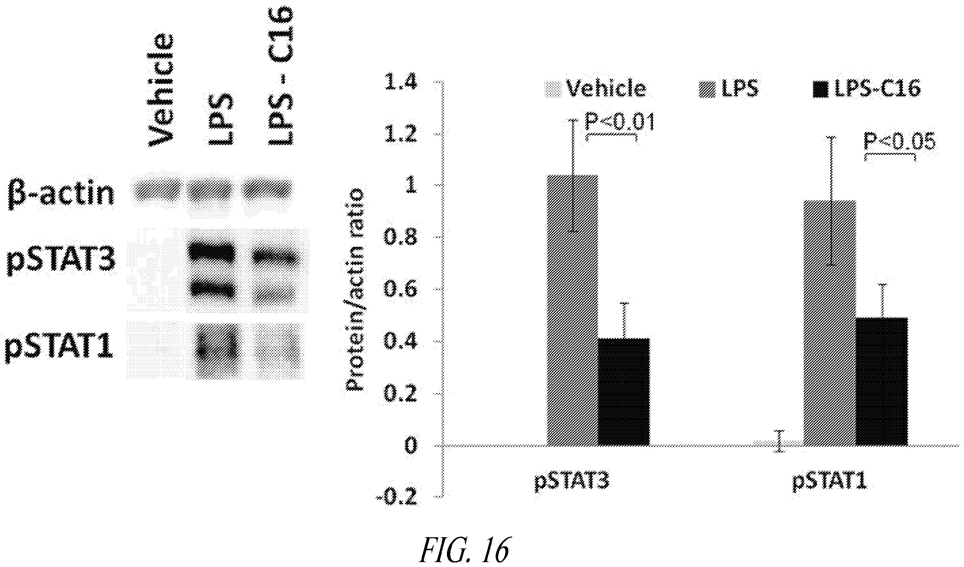

Figure 16 presents data showing that C16 treatment inhibits LPS induced STAT1/3 activation. BV2 cells were treated with either vehicle or Lps (1µg/ml) or Lps (1µg/ml) with C16 (1µM) for 24 hours. Total protein was harvested and subjected to western blot assay. β-actin expression was used for loading control (means ± SE).

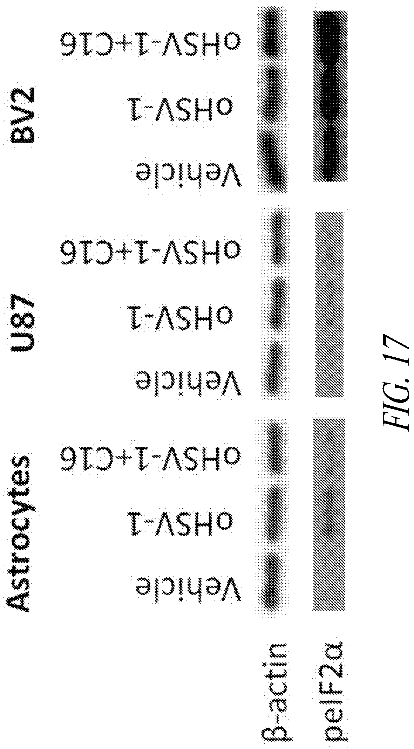

Figure 17 presents data showing that eIF2α phosphorylation inhibition effect of C16 in different cell types infected with oHSV-1. Indicated cells were pre-treated with vehicle or 1 µM C16 for 2 hours before the infection with G207 at an MOI of 1. Total protein was extracted at 24 hours post-treatment and subjected to western blot analysis.

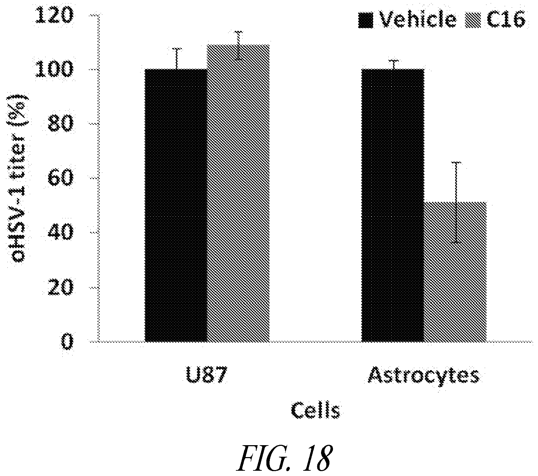

Figure 18 presents data showing the effect of C16 on G207 replication in different cell types. Indicated cells were pre-treated with vehicle or 1 µM C16 for 2 hours before the infection with oHSV-1 (G207) at an MOI of 1. Total protein was extracted at 24 hours.

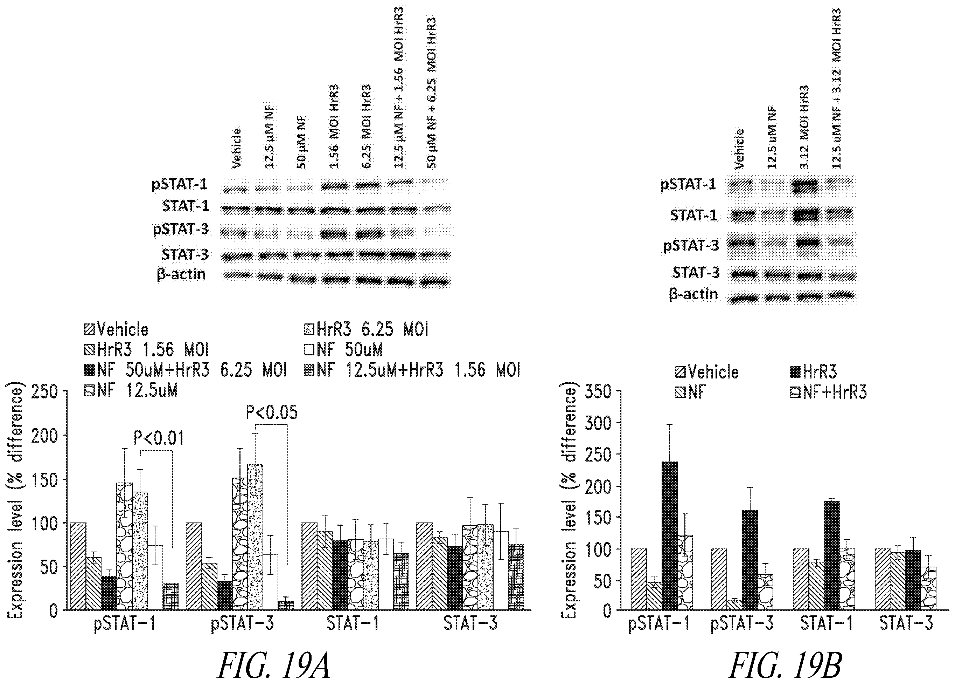

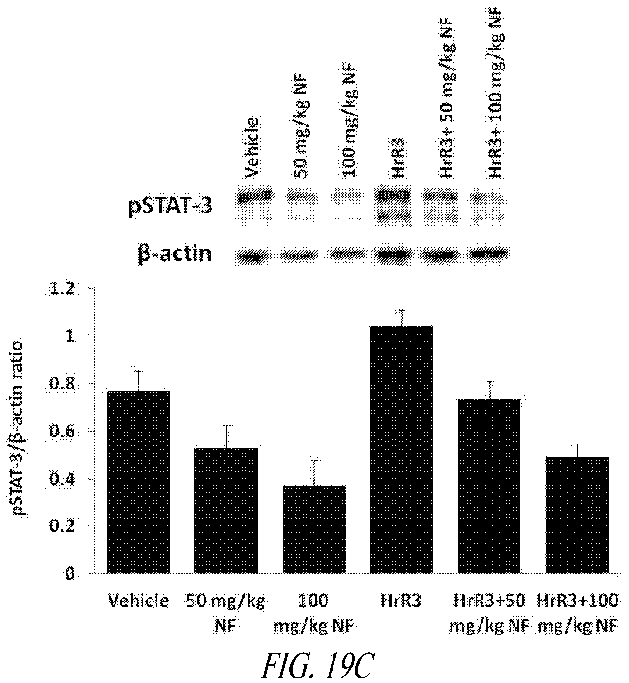

Figures 19A-C show STAT inhibition by the combination of NF and oHSV-1. Fig. 19A and Fig. 19B, U87 cells (A) or CT26 cells (B) were treated with the indicated concentrations of HrR3 or NF or a combination. Total protein was extracted after 24 hours and subjected to western blot analysis. Band intensity was measured using Image J software and normalized to β-actin. Samples were run in triplicate and data are as means ± S.E. Statistically significant differences are indicated by P<0.05 or P<0.01. Fig. 19C, Mice subcutaneously bearing U87 tumors were intratumorally injected with a single dose of HrR3 virus (1X10^6 PFU) and/or peritoneally injected doses of NF as indicated. Tumors were harvested on day 10 post treatment and total protein was extracted from a mouse of each treatment group and subjected to western blot analysis in triplicate. Results are reported as means ± S.E.

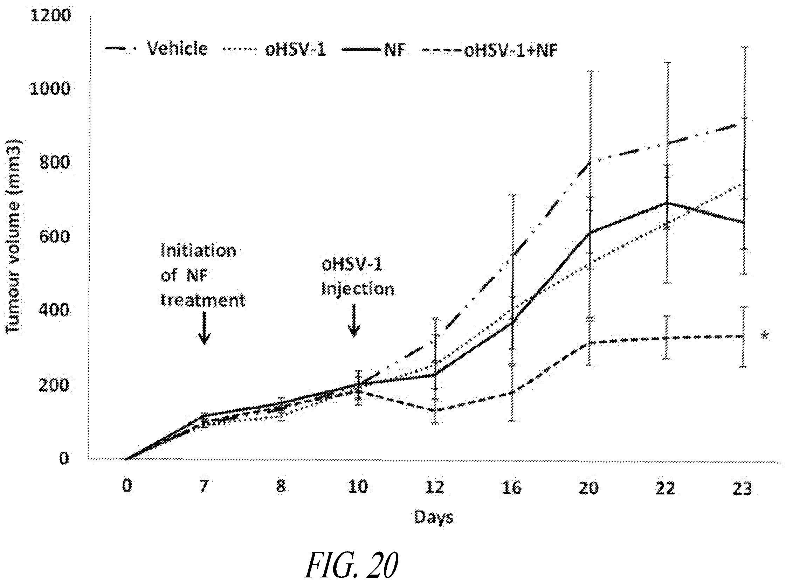

Figure 20 shows the enhanced anti-tumour effect of the NF and oHSV-1 combination in-vivo. CT26 (colon cancer) tumour bearing BALB/C mice were treated with a single dose of HrR3 (oHSV-1) virus (2X10^7 PFU) or vehicle and given daily peritoneal injections of 50mg/kg NF alone or in combination with HrR3 virus (5 mice in each group).Tumor sizes were measured using calipers (length X height X width /2). Data are the means ± S.E. and statistically significance differences between treatment with the NF and oHSV-1 combination, NF alone, and oHSV-1 alone are indicated by the p value, * P<0.05.

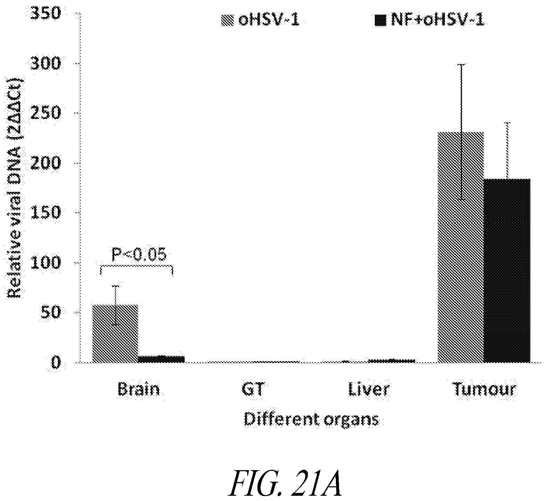

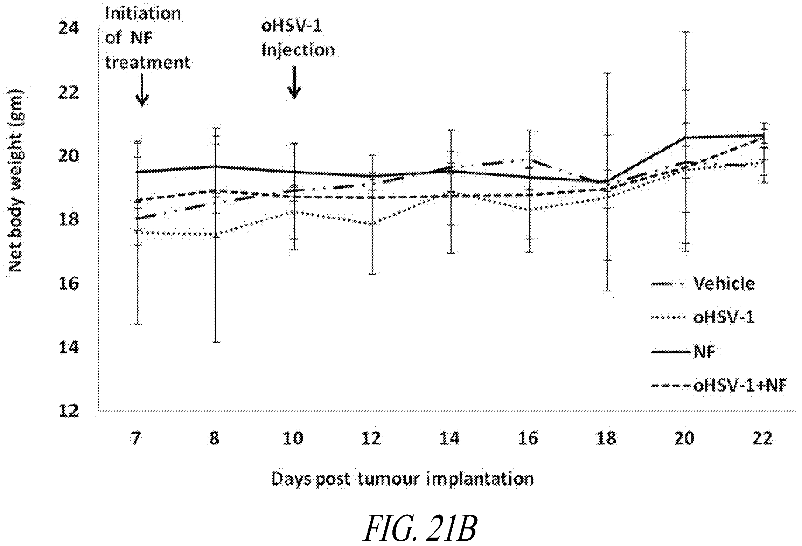

Figures 21A-B demonstrate the safety of NF and oHSV-1 combination in-vivo. Fig. 21A, total genomic DNA was extracted from the harvested tumours and other organs (n=2). Viral DNA (ICP27) was detected by qPCR and normalized to β-actin. Samples were run in quadruplicate and data are the averages of 2 mice ±S.E. Fig. 21B, CT26 (colon cancer) tumour bearing BALB/C mice were treated with NF or HrR3 alone or in combination as described in the material and method. Body weight was measured using a measuring scale and tumour weight was calculated from the tumour volume (1mm3 = 1 gm). Net body weight was obtained by subtracting tumour weight from the body weight. Data represents the mean of net body weight of three randomly selected mice from each group ±S.E.

### DETAILED DESCRIPTION

The present invention may be understood more readily by reference to the following detailed description of preferred embodiments of the invention and the Examples included herein.

The present disclosure provides methods, compositions, and kits for administering both an oncolytic HSV (oHSV) and a STAT1/3 phosphorylation inhibitor, such as a nitrofuran, or C16 or analogues or derivatives thereof, to , to cancer cells, typically in vivo to a subject in need of cancer treatment. The term "cancer," as used herein, refers to a cancer of any kind and origin, including tumor-forming cells, blood cancers, and transformed cells. The term "cancer cell," as used herein, includes cancer or tumor-forming cells, transformed cells, or a cell that is susceptible to becoming a cancer or tumor-forming cell. Representative forms of cancer include carcinomas, sarcomas, myelomas, leukemia's, lymphomas, and mixed types of the above. Further examples include, but are not limited to those discussed in more detail below.

Administration of oHSV and a STAT1/3 phosphorylation inhibitor results in more tumor cell death than administration of either oHSV or a STAT1/3 phosphorylation inhibitor alone. Moreover, the combination treatment is synergistic, allowing smaller doses of both the inhibitor and the virus. Smaller doses confers many advantages, such as lessening the morbidity associated with each agent and decreased costs.

### A. STAT1/3 phosphorylation inhibitors

Signal Transducer and Activator of Transcription (STAT) proteins are a family of cytoplasmic transcription factors consisting of 7 members, including STAT1 and STAT3. They play crucial roles in regulating a number of diverse biological functions including cell proliferation, differentiation, apoptosis, inflammatory response, immunity, and angiogenesis. STAT1/3 regulates diverse biological functions including cell growth, differentiation, and apoptosis. In addition, STAT1/3 plays a key role in regulating host immune and inflammatory responses and in the pathogenesis of many cancers. The STAT1/3 signaling pathway is constitutively activated in many cancers and has been the target for development of STAT1/3 inhibitors to treat cancers.

In general, STATs are activated by tyrosine kinases, including receptor associated tyrosine kinases (e.g., Janus kinases (JAKs)), by receptors with intrinsic tyrosine kinase activity (e.g. PDGFR, EGFR, FLT3), and non-receptor protein tyrosine kinases (PTKs) (e.g., c-Src Bcr-Abl, and Brk (Breast tumor kinase)).

Many different STAT1/3 inhibitors are known. Examples of inhibitors may be found in e.g., Furqan et al. (J of Hemat. & Oncology, 6:90, 2013; incorporated in its entirety). A variety of commercial sources sell STAT1/3 inhibitors (e.g., R&D Systems, MN, USA; InvivoGen, CA USA). Additional inhibitors may be identified using various assays (e.g., Szelag et al., PLOSOne, http://dx.doi.org/10.1371/journal.pone.0116688).

Examples of two particular STAT1/3 inhibitors that are useful in the compositions disclosed herein are nifuroxazide and C16.

### 1. Nitrofurans

Nitrofurans are a class of drugs characterized by a having a furan ring and a nitro group. This class of drugs includes a wide variety of antibacterial drugs (e.g., Difurazone, Furazolidone, Nifturfoline, Nifuroxazide, Nifurquinazol, Nifurtoinol, Nifurzide, Nitrofural (also referred to as nitrofurazone), Nitrofurantoin and Ranbezolid) and antimicrobial drugs (e.g., Furaltadone, Furazidine, Furylfuramide, Nifuratel, andd Nifurtimox.

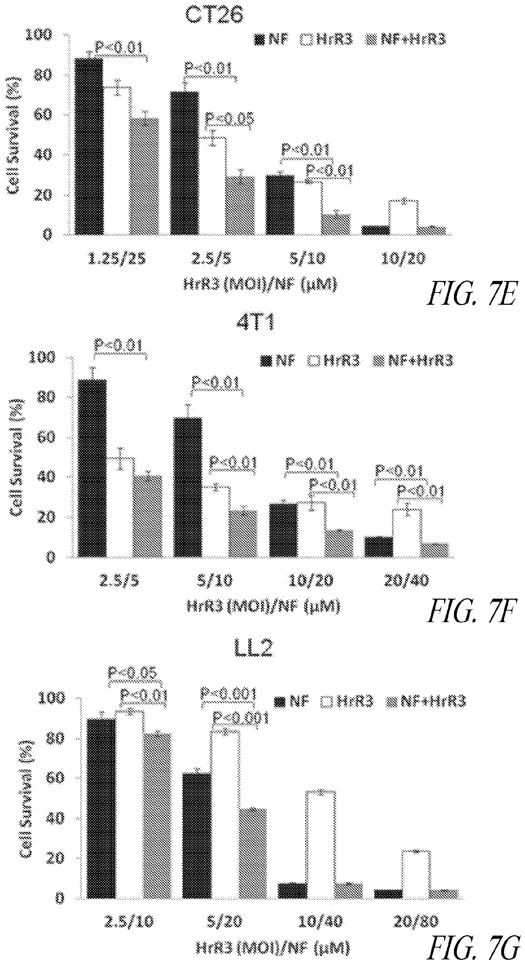

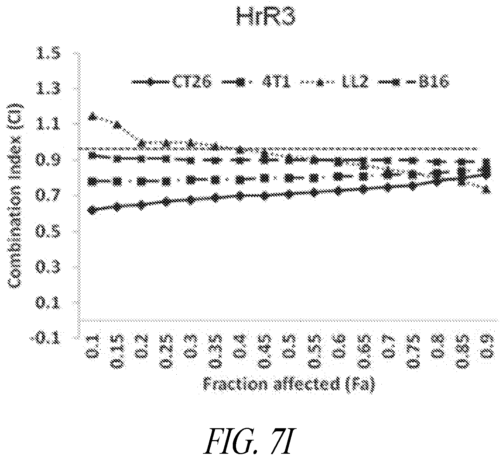

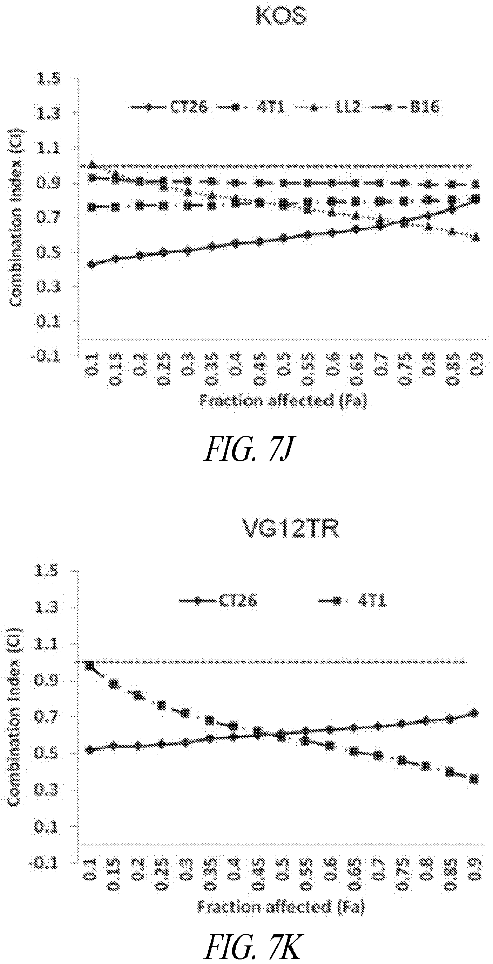

One particularly suitable drug for use within the present invention is Nifuroxazide (Benzoic acid, 4-hydroxy-, [(5-nitro-2-furanyl)metylene]hydrazide, CAS 965-52-6, Fig. 7), which is often used as an oral antibiotic and has been used to treat colitis and diarrhea in humans and non-human animals.

Nifuroxazide has been reported as a potent STAT1/3 signaling pathway inhibitor. As demonstrated in the Examples, nifuroxazide is active against tumor cells in vitro and in vivo, as well as enhancing replication efficiency of oHSV in tumor cells. Nifuroxazide is commercially available from a number of vendors.

Nifuroxazide derivatives and analogues may also be used. Derivatives include heterocyclic compounds, such as 5-nitro-heterocyclic nifuroxazide. Analogues include substitutions of the nitrofuran group by e.g. benzofuroxan and its derivatives having different substitutions on the benzyl ring (Fraias et al. BMC Cancer 15:807, 2015, incorporated in its entirety), substituted benzoic acids (Tavares et al. Boll Chim Farm 136:244, 1997; Masunari and Tavales, Bioorganic & Med Chem 15:4229, 2007; incorporated in their entirety). In addition, various salt forms and prodrugs may be used.

### 2. C16, and analogues and derivatives

C16 is a drug that acts as a selective inhibitor of the enzyme double-stranded RNA-dependent protein kinase (PKR) and also inhibits phosphorylation of STAT 1/3. C16 is also known as PKRi. C16 is an imidazolo-oxindole derivative that has a chemical formula of 6,8-Dihydro-8-(1H-imidazol-5-ylmethylene)-7H-pyrrolo[2,3-g]benzothiazol-7-one. The chemical structure of C16 is shown below. Within the context of the disclosure, C16 also includes salt forms, prodrugs, and derivatives thereof.

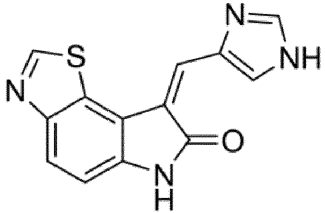

### B. Oncolytic Virus

An oncolytic virus is a virus that will lyse cancer cells (oncolysis), preferably in a selective manner. The term "oncolytic," as used herein, refers to a tumor selective replicating virus, such as a human herpesvirus, which induces cell death in the infected cancer cell and/or tissue. Although normal or non-tumor cells may be infected, cancer cells are infected and selectively undergo cell death, in comparison to the normal or non-cancer cells of a subject. "Cell death," as used herein, includes all forms of cell death, including for example cell lysis and/or apoptosis. Viruses that selectively replicate in dividing cells over non-dividing cells are often oncolytic. Oncolytic viruses suitable for use herein include Herpes Simplex Viruses, adenoviruses, coxsackie, measles viruses, Newcastle disease viruses, parvoviruses, polioviruses, reoviruses, Seneca Valley virus, retroviruses, vaccinia viruses, tanapoxviruses, vesicular stomatitis viruses, myxoma viruses and influenza A viruses.

Oncolytic viruses suitable for the compositions and methods described herein may comprise wild-type viruses, replicating viruses and modified replication-competent derivatives thereof and non-replicating viruses, CPG-armed viruses, as well as related viruses or vectors based on such viruses or derivatives. Generally, replication competent viruses will be used, such that treatment of a subject may be achieved by killing of the cancer cells, i.e., the cancer cells are killed by the oncolytic and cytotoxic activity of the oncolytic virus. The oncolytic virus included in the compositions, and used in the methods, described herein may constitute active forms of such viruses or, alternatively, viral vectors that are configured to encode and produce the desired oncolytic virus in a targeted cancer cell. The term "viral vector," as used herein, refers to a nucleic acid molecule that is used as a vehicle to deliver one or more nucleic acid molecules into a cell to allow recombination. The viral vector may be a plasmid construct that encodes and is used to generate an oncolytic virus (such as a human herpesvirus) in a cancer cell - or it may be an oncolytic virus genome (e.g., a non-recombined human herpesvirus genome).

Herpes Simplex Virus (HSV) 1 and 2 are members of the Herpesviridae family, which infect humans. The HSV genome contains two unique regions, which are designated unique long (U_{L}) and unique short (U_{S}) region. Each of these regions is flanked by a pair of inverted terminal repeat sequences. There are about 75 known open reading frames. The viral genome has been engineered to develop oncolytic viruses for use in e.g. cancer therapy. Tumor-selective replication of HSV is conferred by mutation of the HSV ICP34.5 (also called γ34.5) gene. HSV contains two copies of ICP34.5. Mutants inactivating one or both copies of the ICP34.5 gene are known to lack neurovirulence, i.e. be avirulent/ non-neurovirulent and be oncolytic.

In some embodiments, the oHSV has one or both of the γ34.5 genes modified such that it is incapable of expressing a functional ICP34.5 protein. The genes may be modified by mutation of one or more nucleotides, insertions, deletions, substitutions, etc. The alteration may be in the coding sequence, non-coding sequence (e.g., promoter) or both. In some embodiments, both copies of the γ34.5 genes are mutated.

The oHSV may have additional mutations, which may include disabling mutations e.g., deletions, substitutions, insertions), which may affect the virulence of the virus or its ability to replicate. For example, mutations may be made in any one or more of ICP6, ICPO, ICP4, ICP27, ICP47, ICP 24, ICP56. Preferably, a mutation in one of these genes (optionally in both copies of the gene where appropriate) leads to an inability (or reduction of the ability) of the HSV to express the corresponding functional polypeptide. In some embodiments, the promoter of a viral gene may be substituted with a promoter that is selectively active in target cells or inducible.

The oHSV may also have genes and nucleotide sequences that are non-HSV in origin. For example, the oHSV may comprise a sequence that encodes a prodrug, a sequence that encodes a cytokine or other immune stimulating factor, a tumor-specific promoter, an inducible promoter, an enhancer, a sequence homologous to a host cell, among others.

Suitable oncolytic HSV may be derived from either HSV-1 or HSV-2, including any laboratory strain or clinical isolate. In some embodiments, the oHSV may be or may be derived from one of laboratory strains HSV-1 strain 17, HSV-1 strain F, or HSV-2 strain HG52. In other embodiments, it may be of or derived from non-laboratory strain JS-1. Suitable HSV vectors include those taught in US 7,223,593, US 7,537,924, US 7,063,835, US 7,063,851, US 7,118,755, US 8,277,818, and US 8,680,068. Other suitable HSV-1 viruses are in the table below. Within certain embodiments of the invention, the oncolytic virus is a HSV-1 with a defective viral ribonuclease reductase gene, and optionally an otherwise intact ICP34.5 gene. Particularly preferred HSV-1 mutants in this regard include hrR3 (see Kulu et al, Cancer Gene Therapy (2009) 16, 291-297; doi:10.1038/cgt.2008.83; published online 7 November 2008; see also Goldstein DJ, Weller SK. Herpes simplex virus type 1-induced ribonucleotide reductase activity is dispensable for virus growth and DNA synthesis: isolation and characterization of an ICP6 lacZ insertion mutant. J Virol 1988; 62: 196-205).

A number of oncolytic viruses are known in the art. Examples include :

| Virus | Name | references |
|---|---|---|
| HSV-1 | HrR3 | [1] |
| | G2O7 | [2, 3] |
| | G47Delta | [4] |
| | HSV 1716 | [5, 6] |
| | HF10 | [7] |
| | NV1020 | [8] |
| | T-VEC | [9] |
| | J100 | [10] |
| | M002 | [11] |
| | NV1042 | [12] |
| | G2O7-IL2 | [13] |
| | rQNestin34.5 | [14] |
| | G47Δ-mlL-18 | [15] |
| Adenovirus | H101 | [16] |
| | Onyx-015 | [17] |
| | CV706 | [18] |
| | CG0070 | [19] |
| | Telomelysin | [20] |
| | Ad5-CD/TKrep | [21] |
| | Ad5-D24-RGD | [22] |
| | CGTG-102 | [23] |
| | INGN-007 | [24] |
| | ColoAd1 | [25] |
| | CG787 | [26] |
| | H103 | [27] |
| Coxsackie | CAVATAK | [28] |
| Measles virus | MV-CEA | [29] |
| | MV-NIS | [30] |
| | MV-aPD-L1 | [31] |
| | MV-aCTLA-4 | [31] |
| | MV GM-CSF | [32] |
| Newcastle disease virus | NDV-HUJ | [33] |
| | PV701 | [34] |
| | MTH-68/H | [35] |
| | rNDV/F3aa-GM-CSF | [36] |
| | rNDV/F3aa-IFN-γ | [36] |
| | rNDV/F3aa-TNF-α | [36] |
| | rNDV-IL2 | [37] |
| | rNDV/F3aa-IL-2 | [36] |
| Parvovirus | H-1PV | [38] |
| Poliovirus | PVS-RIPO | [39] |
| Reovirus | Reolysin | [40] |
| Seneca Valley virus | NTX-010 | [41] |
| Retrovirus | Toca 511 | [42] |
| Vaccinia | JX-594 | [43] |
| | Dryvax | [44] |
| | JX-795 | [45] |
| | VVLΔTK-IL10 | [46] |
| | rV-4-1BBL | [47] |
| | vvCCL 19 | [48] |
| | OVV-CXCR4-A-Fc | [49] |
| | vvDD-CDSR | [50] |
| | GL-ONC1 | [51] |
| Tanapoxvirus | TPV/Δ66R/fliC | [52] |
| | TPV/Δ66R/mMCP-1 | [52] |
| Vesicular stomatitis virus | rVSV-IL12 | [53] |
| | opt.hlL-15 | [54] |
| | rrVSV | [55] |
| Myxoma virus | vMyx-GFP | [56] |
| | vMyx-IL15Rα-tdTr | [57] |
| Influenza A viruses | deINS1-IL-15 | [58] |

### C. Therapeutic compositions

Therapeutic compositions are provided that may be used to prevent, treat, or ameliorate the effects of a cancer. More particularly, some therapeutic compositions comprise an oncolytic virus as described herein. Within preferred embodiments, the therapeutic composition can comprise an oncolytic virus as described herein. Within preferred embodiments, the therapeutic composition can comprise an oncolytic virus and a STAT 1/3 phosphorylation inhibitor (such as C16 or a nitrofuran) or a PKR inhibitor (e.g., C16), and a pharmaceutically acceptable carrier.

The combination of an oncolytic virus with a STAT 1/3 phosphorylation inhibitor (such as C16, or an analogue or derivative, or a nitrofuran) or a PKR inhibitor (e.g., C16 or an analogue or derivative) is particularly efficacious in preventing, treating, and/or ameliorating the effects of cancer. More specifically, and as demonstrated in the Examples below, the invention provides that the co-administration of a STAT 1/3 phosphorylation inhibitor (such as C16 and a nitrofuran), along with an oncolytic virus, is effective to prevent macrophage and microglia inhibition of oncolytic viral activity, thereby enhancing the efficacy of the co-delivered oncolytic virus. The oncolytic virus may be any of a variety of oncolytic viruses, including those described above, and will frequently be a Herpes Simplex Virus I (oHSV-1) described herein.

In certain embodiments, the compositions will further comprise a pharmaceutically acceptable carrier. The phrase "pharmaceutically acceptable carrier" is meant to encompass any carrier, diluent or excipient that does not interfere with the effectiveness of the biological activity of the oncolytic virus and that is not toxic to the subject to whom it is administered (see generally Remington: The Science and Practice of Pharmacy, Lippincott Williams & Wilkins; 21st ed. (May 1, 2005 and in The United States PharmacopE1A: The National Formulary (USP 40 - NF 35 and Supplements).

In the case of an oncolytic virus (or viral vectors encoding the same), non-limiting examples of suitable pharmaceutical carriers include phosphate buffered saline solutions, water, emulsions (such as oil / water emulsions), various types of wetting agents, sterile solutions, and others. Additional pharmaceutically acceptable carriers include gels, bioadsorbable matrix materials, implantation elements containing the oncolytic virus, or any other suitable vehicle, delivery or dispensing means or material(s). Such carriers can be formulated by conventional methods and can be administered to the subject at an effective dose. Additional pharmaceutically acceptable excipients include, but are not limited to, water, saline, polyethyleneglycol, hyaluronic acid and ethanol. Pharmaceutically acceptable salts can also be included therein, e.g., mineral acid salts (such as hydrochlorides, hydrobromides, phosphates, sulfates, and the like) and the salts of organic acids (such as acetates, propionates, malonates, benzoates, and the like).. Such pharmaceutically acceptable (pharmaceutical-grade) carriers, diluents and excipients that may be used to deliver the STAT1/3 inhibitor to a target cancer cell will preferably not induce an immune response in the individual (subject) receiving the composition (and will preferably be administered without undue toxicity).

In the case of C16 or nitrofuran (both a small molecule), non-limiting examples of suitable pharmaceutical acceptable carriers may include carriers, diluents and adjuvants, such as Dulbecco's phosphate buffered saline, pH about 7.4; 0.9% saline (0.9% w/v NaCl); and 5% (w/v) dextrose.

The compositions provided herein can be provided at a variety of concentrations. For example, dosages of oncolytic virus can be provided which ranges from a dose of greater than about 10⁹ plaque forming units ("pfu"), from between about 10² to above 10⁹ pfu, between about 10² to about 10⁷ pfu, between about 10³ to about 10⁶ pfu, or between about 10⁴ to about 10⁵ pfu. Within certain embodiments (and utilizing oncolytic HSV as an example), dosage forms for humans can range from about 10⁶ to about 10⁹ pfu. Within further embodiments, the dosage form can range from about 10⁶ to about 10⁸ pfu/ml, with up to 4 mls being injected into a patient with large lesions (e.g., >5 cm) and smaller amounts (e.g, up to 0.1mls) in patients with small lesions (e.g., < 0.5 cm) every 2 - 3 weeks, of treatment.

Similarly, a variety of dosage forms for C16 (or analogues or derivatives) and nitrofurans can readily be provided based upon standard prescribing regimens (see e.g., Physician's Desk Reference, 71st ed., PDR Staff, 2017; and The Merck Manual, 19th ed., Robert S. Porter, 2011).

Within certain embodiments of the invention, due to the synergsym of the oncolytic virus and the C16 (or analogue or derivative) and nitrofuran, lower dosages than standard may be utilized. Hence, within certain embodiments less than about 10⁶ pfu/ml (with up to 4 mls being injected into a patient every 2 - 3 weeks) can be administered to a patient, along with (either sequentially or simultaneously, C16 (or an analogue or derivative thereof) or a nitrofuran.

The compositions may be stored at a temperature conducive to stable shelf-life, and includes room temperature (about 20C), 4C, -20C, -80C, and in liquid N2. Because compositions intended for use in vivo generally don't have preservatives, storage will generally be at colder temperatures. Compositions may be stored dry (e.g., lyophilized) or in liquid form.

### D. Administration

In addition to the compositions described herein, various methods of using such compositions to treat or ameliorate cancer are provided. Within preferred embodiments of the invention methods are provided for improving the efficacy of an oncolytic virotherapy, which comprises the steps of (1) administering an oncolytic virus to a subject and (2) administering a STAT 1/3 phosphorylation inhibitor (such as C16, or an analogue or derivative thereof, or a nitrofuran) in an amount that is effective to reduce microglia- or macrophage-mediated suppression of replication of the oncolytic virus in a cancer cell. In such embodiments, an effective dose of the (1) oncolytic virus and (2) STAT 1/3 phosphorylation inhibitor is typically delivered to the subject. Within various embodiments of the invention steps (1) and (2) may be completed in either order, or, at the same time.

The terms "effective dose" and "effective amount" refers to amounts of the oncolytic virus and inhibitor of STAT 1/3 phosphorylation or PRK that are sufficient to effect treatment of a targeted cancer, e.g., amounts that are effective to reduce a targeted tumor size or load, or otherwise hinder the growth rate of targeted tumor cells. More particularly, such terms refer to amounts of oncolytic virus and a inhibitor of STAT 1/3 phosphorylation or PRK that are effective, at the necessary dosages and periods of treatment, to achieve a desired result. For example, in the context of treating a cancer, an effective amount of the compositions described herein is an amount that induces remission, reduces tumor burden, and/or prevents tumor spread or growth compared to the response obtained without administration of the oncolytic virus and PKR inhibitor described herein. Effective amounts may vary according to factors such as the subject's disease state, age, gender, and weight, as well as the pharmaceutical formulation, the route of administration, and the like, but can nevertheless be routinely determined by one skilled in the art.

The therapeutic compositions are administered to a subject diagnosed with cancer or is suspected of having a cancer. Subjects may be human or non-human animals.

The compositions are used to treat cancer. The terms "treat" or "treating" or "treatment," as used herein, means an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, diminishment of the reoccurrence of disease, and remission (whether partial or total), whether detectable or undetectable. The terms "treating" and "treatment" can also mean prolonging survival as compared to expected survival if not receiving treatment.

Representative forms of cancer include carcinomas, leukemia's, lymphomas, myelomas and sarcomas. Further examples include, but are not limited to cancer of the bile duct cancer, brain (e.g., glioblastoma), breast, cervix, colorectal, CNS (e.g., acoustic neuroma, astrocytoma, craniopharyogioma, ependymoma, glioblastoma, hemangioblastoma, medulloblastoma, menangioma, neuroblastoma, oligodendroglioma, pinealoma and retinoblastoma), endometrial lining, hematopoietic cells (e.g., leukemia's and lymphomas), kidney, larynx, lung, liver, oral cavity, ovaries, pancreas, prostate, skin (e.g., melanoma and squamous cell carcinoma) and thyroid. Cancers can comprise solid tumors (e.g., sarcomas such as fibrosarcoma, myxosarcoma, liposarcoma, chondrosarcoma and osteogenic sarcoma), be diffuse (e.g., leukemia's), or some combination of these (e.g., a metastatic cancer having both solid tumors and disseminated or diffuse cancer cells).

Benign tumors and other conditions of unwanted cell proliferation may also be treated.

At its most basic, oHSV and a STAT1/3 inhibitor are co-administered to a subject. Co-administration may be simultaneous or sequential. When sequential, either oHSV or the STAT1/3 inhibitor may be given first in time. The means of administration of each agent may be the same (e.g., injection) or different (e.g., one is given orally and the other by injection), regardless of whether they are administered simultaneously or sequentially. Multiple administrations of one or both of the agents may be given to the subject. For example, a subject may receive one initial dose of both oHSV and inhibitor and a second dose of just inhibitor.

The oncolytic virus and the STAT1/3 inhibitor may be given by a route that is e.g. oral, topical, parenteral, systemic, intravenous, intramuscular, intraocular, intrathecal, intratumor, subcutaneous, or transdermal. Within certain embodiments the oncolytic virus and/or STAT1/3 inhibitor may be delivered by a cannula, by a catheter, or by direct injection. The site of administration may be intra-tumor or at a site distant from the tumor. The route of administration will often depend on the type of cancer being targeted.

The optimal or appropriate dosage regimen of the oncolytic virus (and PKR inhibitor / STAT 1/3 phosphorylation inhibitor) is readily determinable within the skill of the art, by the attending physician based on patient data, patient observations, and various clinical factors, including for example a subject's size, body surface area, age, gender, and the particular oncolytic virus being administered, the time and route of administration, the type of cancer being treated, the general health of the patient, and other drug therapies to which the patient is being subjected. According to certain embodiments, treatment of a subject using the oncolytic virus and a PKR inhibitor and/or STAT 1/3 phosphorylation inhibitor described herein may be combined with additional types of therapy, such as chemotherapy using, e.g., a chemotherapeutic agent such as etoposide, ifosfamide, adriamycin, vincristin, doxicyclin, and others.

oHSV and the STAT1/3 inhibitor may be formulated as medicaments and pharmaceutical compositions for clinical use and may be combined with a pharmaceutically acceptable carrier, diluent, excipient or adjuvant. The formulation will depend, at least in part, on the route of administration. Suitable formulations may comprise the virus and inhibitor in a sterile medium. The formulations can be fluid, gel, paste or solid forms. Formulations may be provided to a subject or medical professional

A therapeutically effective amount is preferably administered. This is an amount that is sufficient to show benefit to the subject. The actual amount administered and time-course of administration will depend at least in part on the nature of the cancer, the condition of the subject, site of delivery, and other factors.

In addition, in certain embodiments, a method is provided for improving oncolytic activity in a cancer cell (and for preventing macrophage and microglia inhibition of oncolytic viral activity in a cancer cell), which comprises the steps of (a) administering an oncolytic virus to a cancer cell and (b) administering a STAT 1/3 phosphorylation inhibitor in an amount that is effective to reduce such microglia- or macrophage-mediated suppression of replication of the oncolytic virus in the targeted cancer cell, such as a glioblastoma cell.

In the methods described herein, the oncolytic virus and PKR inhibitor and/or STAT 1/3 phosphorylation inhibitor may be administered to a cancer cell (or subject) together in a single formulation or, alternatively, in series as separate formulations.

Within yet other embodiments of the invention the oncolytic virus and PKR inhibitor and/or STAT 1/3 phosphorylation inhibitor can be administered intratumorally, or, after surgical resection of a tumor.

The following examples are offered by way of illustration, and not by way of limitation.

### EXAMPLES

### EXAMPLE 1

### Nifuroxazide Possesses Potent Anti-Tumor Effect

In this example, the antitumor effect of nifuroxazide is evaluated in different tumor cells. Cells were treated with nifuroxazide for 72 hours (Figure 1A). Inhibitory concentration 50% (IC50) of nifuroxazide against BTUC, 9L, SF126, U87, U373, LnCap and MCF7 cells were approximately 15 µM, 27 µM, 20 µM, 20 µM, 23 µM, 5 µM and 20 µM respectively. To verify the anti-tumor efficacy of nifuroxazide in vivo, animals with subcutaneous U87 tumor implants were injected in the peritoneum with vehicle, 1 mg/kg nifuroxazide and 50 mg/kg nifuroxazide. Significant tumor regression effect was observed in nifuroxazide-treated animals. Tumor volume was reduced around 25% and 60% after 7 days treatment with 1 mg/kg nifuroxazide and 50 mg/kg nifuroxazide respectively (Figure 1B).

### EXAMPLE 2

### NIFUROXAZIDE ENHANCES OHSV-1 REPLICATION EFFICIENCY IN TUMOR CELLS

In this example, the effect of nifuroxazide on oHSV-1 anti-tumor efficacy was evaluated. oHSV-1 infected treated glioma cells were incubated with low concentration of nifuroxazide. Replication increased 27- and 40-fold with 0.5 µM nifuroxazide and 10 µM nifuroxazide treated U87 cells respectively. Similarly, virus replication increased 0.8- and 4.7-fold virus in 9L cells treated with 0.5 µM nifuroxazide and 10 µM nifuroxazide respectively (Figure 2A). PCR viral titer also confirms the dose dependent HrR3 replication enhancement in U87 glioma cells but not in normal glial cells (Figure 2B).

### EXAMPLE 3

### NIFUROXAZIDE AND OHSV-1 PROVIDE SYNERGISTIC ANTI-TUMOR EFFECT.

In this example, the combination anti-tumor effect of nifuroxazide and oHSV-1 is evaluated. HrR3 is an oncolytic HSV-1[59, 60], with a mutation /deletion of ICP6 gene [59]. Ribonucleotide reductase (RR) is essential for the synthesis of deoxyribonucleotides, which is needed for viral DNA synthesis and replication and it is encoded by the viral ICP6 gene. Increased expression of mammalian RR is found in most of the rapidly dividing cells and RR deletion mutants (HrR3) replicate efficiently only in the cells that compensate for the loss of ICP6 by expressing the mammalian complement of RR [61].

The individual and combined cytotoxic effect of nifuroxazide and HrR3 was determined in U87 glioma cells. IC₅₀ of nifuroxazide and HrR3 individual dose against U87 cells were around 20 µM and 3.12 MOI respectively. While, in combination approximately 4 µM nifuroxazide and MOI of 1 HrR3 dose provide ICso against U87 cells (Figure 3A). To ask whether this combination provide synergistic anti-tumor effect, data were analyzed by calcusyn software (http://www.biosoft.com/w/calcusyn.htm). 0.78/3.12, 1.56/6.25, 3.12/12.5, 6.25/25 and 25/100 HrR3/nifuroxazide doses were demonstrated to be synergistic (Figure 3B).

### EXAMPLE 4

### NIFUROXAZIDE INCREASES OHSV-1 IMMEDIATE EARLY GENE, ICP27 EXPRESSION

In this example, the underlying mechanism of nifuroxazide mediated oHSV-1 replication enhancement was examined. The effect of nifuroxazide on viral immediate early gene, ICP27 and ICP4 expression was established by Western blot analysis. Low concentration of nifuroxazide (0.5 µM, 1 µM and 10 µM) significantly increases ICP27 expression, but not ICP4 expression (Figure 4A). qRT-PCR analysis further verify the nifuroxazide mediated up-regulation of ICP27 mRNA expression (Figure 4B).

### EXAMPLE 5

### NIFUROXAZIDE REDUCES STATS ACTIVATION IN OHSV-1 INFECTED TUMOR CELLS

In this example, the cellular factor responsible for the synergistic effect was evaluated. Expression of key regulator of the Type1 interferon signaling, STAT1 and STAT3, was determined in nifuroxazide treated oHSV-1 infected U87 cells. Nifuroxazide mediated dose dependent STAT1 and STAT3 phosphorylation reduction were observed in U87 cells (Figure 5A). To verify whether specific STAT3 inhibitor (Stattic) also provide similar additive effect with oHSV-1, an MTT assay was used to determine the combination anti-tumor efficacy of static and oHSV-1. Like a combination of nifuroxazide and HrR3, a combination of stattic and HrR3 also showed additive anti-tumor effect against U87 cells (Figure 5B).

### EXAMPLE 6

### DOSE DEPENDENT OHSV-1 AMPLIFICATION IN GLIOBLASTOMA TUMORS IN-VIVO

In this example, oHSV-1 replication augmentation effect was evaluated in vivo. Subcutaneously implanted U87 tumors were treated with either HrR3 alone or in combination with 50mg/kg nifuroxazide or 100mg/kg nifuroxazide. Dose dependent oHSV-1 amplification was determined by measuring viral DNA by qPCR in tumor mass. oHSV-1 augmentation was observed 1.6 fold and 2.2 fold by 50mg/kg nifuroxazide 100mg/kg nifuroxazide respectively (Figure 6A). Nifuroxazide mediated oHSV-1 amplification was also confirmed by immunohistochemistry assay in harvested tumor tissues (Figure 6B).

### EXAMPLE 7

### SYNERGISTIC EFFECT OF NIFUROXAZIDE AND OHSV-1 ON TUMOR CELLS

In this example, the effect of nifuroxazide and oHSV-1 on tumor cells was evaluated. A variety of cancer cell types were treated with nifuroxazide (from 0-100 µM) or HrR3 virus at a MOI from 0 to 12.5 or KOS virus at a MOI from 1 to 12.5. Cell survival was measured. As shown in Fig. 7A-7C, treatment with nifuroxazide caused cell mortality in all cell lines at higher doses (CT26 (colorectal colon carcinoma), LL2 (Murine Lewis lung carcinoma), U87 (human glioma; B16, murine melanoma; 4T1, murine mammary carcinoma)). All cell lines, except B16, were depleted by both KOS and HrR3 viruses.

Tumor cells were treated with nifuroxazide or medium. Following incubation, virus was added and further incubated for 72 (fig. 7D) or 48 hrs (Fig. 7E-7G). Cytotoxicity was measured by an MTT assay. Error bars represent S.D. and statistically significant differences are indicated. Treatment with both nifuroxazide and virus resulted in significantly less cell survival.

If Fig.7H-7K, cells were treated with varying doses of nifuroxazide or the indicated viruses (HrR3, KOS, or VG12TR) alone or in combination for 48 hrs (7J, 7K) or 72 hrs (7H) Cell viability was measured by a MTT assay and combination index (CI) values were calculated using Chour-Talalay analysis. CI was plotted against the affected fraction (Fa). Cl of <1, Cl=1, and Cl>1 represent synergistic, additive, and antagonistic effect respectively. Treatment with both nifuroxazide and virus had a synergistic effect on all cells.

Fig. 7L presents the dose reduction index for a combination of nifuroxazide and HrR3 virus on various tumor cells. The DRI (dose reduction index) determines the magnitude of dose reduction allowed for each drug when given in synergistic combination, as compared with the concentration of a single agent that is needed to achieve the same effect level. As shown in the table, DRI ranges up to 12.9.

### EXAMPLE 8

### MICROGLIA ISOLATION AND CULTURE.

An E18 Sprague Dawley rat was obtained from Charles River Laboratories (Charles River, Wilmington, MA). Rat primary microglia isolation and culture was conducted according to standard protocols. In brief, cortices were isolated from day 18 embryonic E18 Sprague Dawley rat brain. After 30 minutes incubation in trypsin / EDTA (Invitrogen, Canada), harvested tissue was washed with culture medium and minced in the presence of Dnase I (Invitrogen, Canada). The cell suspension was then centrifuged, resuspended in fresh culture medium and plated on 10 cm culture dishes in high confluency. Microglia cells were cultured in Dulbecco's modified eagle's medium (Sigma, Canada) supplemented with 10% fatal bovine serum (Invitrogen, Canada), 1% antibiotics (penicillin and streptomycin) and maintained at 37oC in 5% CO2. Culture medium was changed every 3-4 days. After 7-10 days, microglias were harvested by gently rocking the plate by hand a couple of times. Finally, microglia floating in the supernatant were plated on a poly-L-lysine coated plate. Cell purity was routinely tested by immunocytochemistry staining for ITGAM (1:200; ProSci Incorporated, CA), which is a microglia-specific integrin protein.

### EXAMPLE 9

### CELL CULTURE.

U87 (human GBM) cells and Vero (African green monkey kidney) cells were obtained from American Type Culture Collection (ATCC, Manasas, VA). BV2 (Mouse microglia) cells were kindly provided by the University of Manitoba. All cells were maintained in Dulbecco's modified eagle's medium (DMEM) supplemented with 10% fetal bovine serum (FBS) and 1% antibiotics (penicillin and streptomycin). All cultures were maintained at 37°C in 5% CO2.

### EXAMPLE 10

### VIRUS REPLICATION ASSAY.

G207 virus was obtained from NeuroVir Therapeut Inc. (San Diego, CA). U87 (5X104) cells alone or with the indicated number of microglia cells in co-culture were incubated overnight with complete culture medium (DMEM with 10% FBS and 1% antibiotic). The cells were infected the subsequent day with G207 virus at a multiplicity of infection (MOI) of 1. Virus infection and treatment was maintained in DMEM medium without FBS and antibiotic. Viruses were harvested after 2-4 days post-infection. After three freeze-thaw cycles, viruses were titrated on Vero cells by a standard plaque assay on 12-well plates by triplicates.

### EXAMPLE 11

### DRUGS AND REAGENTS EFFECT ON VIRUS REPLICATION.

U87 (5X104) alone or U87 + microglia co-culture (5X104 + 5X104) or BV2 microglia cells (5X104) alone were seeded into a 24-well plate. After overnight incubation (to allow the cells to attach), cells were pretreated with indicated concentration of NFkB inhibitor, Bay-11 (Santa Cruz, Canada) or an indicated concentration of imidazolo-oxindole derivative C16 (Milipore, Canada) or vehicle for 1-2 hours. The cells were then infected with oHSV-1 (G2O7 or HrR3) virus at a MOI of 1 in presence of chemical inhibitors for another 2 days. The viruses were harvested and titrated in vero cells by plaque forming assay in 12-well plates by triplicates.

### EXAMPLE 12

### WESTERN BLOTS.

Total protein was harvested with sample buffer (125 mM Tris-HCL, 50% Glycerol, 4% Bromophenol blue and 5% 2-mercaptoethanol) and boiled for 5 minutes. Protein samples were subjected to SDS-PAGE (8% gel), transferred to nitrocellulose membranes and blocked in 5% nonfat milk (Bio Rad) in TBS-Tween 20 (TBS-T) for 1 hour at room temperature. The membranes were then incubated with primary antibody against β-actin at 1:1000 (Cell Signaling, Danvers, MA) or anti-STAT1 antibody at 1:1000 (Cell Signaling, Danvers, MA) or anti-STAT3 antibody at 1:1000 (Cell Signaling, Danvers, MA) or anti-phospho STAT1 antibody (Tyr701) at 1:1000 (Cell Signaling, Danvers, MA) or anti-phospho STAT3 antibody (Tyr705) at 1:1500 (Cell Signaling, Danvers, MA) or anti-phosphor-eIF2α antibody (ser51) at 1:1000 (Cell Signaling, Danvers, MA) or anti-ICP27 antibody at 1:1000 (Abcam, Cambridge, MA) or anti-ICP4 antibody at 1:750 (Abcam, Cambridge, MA) for overnight at 4oC. The membranes were washed the next day with TBS-T three times and incubated with corresponding secondary antibody at 1:3000 (Perkim Elmer, Boston, MA), for 1 hour at room temperature. Membranes were washed with TBS-T three times before visualization using ECL reagent (Perkim Elmer, Boston, MA) and VersaDoc imaging system (Bio-Rad). Band density was measured by using ImageJ software (NIH, Bethesda, MD).

### EXAMPLE 13

### RNA EXTRACTION AND RT-PCR

U87 and BV2 microglia cells were infected with oHSV-1 at a MOI of 1. Total RNA was isolated 24 hours post-infection from BV2 or U87 cells using Triazol reagent (Invitrogen, Canada). RT-PCR was performed by using one-step real-time PCR using KAPA SYBR^{®} FAST One-Step qRT-PCR Universal (D-MARK Biosciences, Canada) following the manufacturer's protocol. cDNA was amplified using the primers listed below, the results were expressed as 2-ΔΔCT, and β-actin was used for normalization.

| **Gene** | **Primer** | **SEQ ID** |
|---|---|---|
| ICP4 | forward | SEQ ID NO:1 |
| ICP4 | reverse | SEQ ID NO:2 |
| ICP27 | forward | SEQ ID NO:3 |
| ICP27 | reverse | SEQ ID NO:4 |
| β-actin | forward | SEQ ID NO:5 |
| β-actin | reverse | SEQ ID NO:6 |
| ICP8 | forward | SEQ ID NO:7 |
| ICP8 | reverse | SEQ ID NO:8 |
| GC | forward | SEQ ID NO:9 |
| GC | reverse | SEQ ID NO:10 |
| VP5 | forward | SEQ ID NO:11 |
| VP5 | reverse | SEQ ID NO:12 |

### EXAMPLE 14

### B-GALACTOSIDASE STAINING.

Cells plated onto 8-well chamber slides were infected with G207 virus and mock infected cells were considered as control. After 24 hours post-infection, cells were fixed by using 0.5% glutaraldehyde solution. Fixed cells were washed twice with PBS and then incubated with 1 mg/ml X-gal solution (Sigma, Canada) diluted with X-gal staining solution (5mM K3Fe, 5mM K4Fe and 2mM MgCI2) at 37oC for one hour. Stained cells were then visualized and imaged by using a light microscope.

### EXAMPLE 15

### CELL PROLIFERATION ASSAY.

Cells were seeded in a 96-well plate at a density of 1 X 104 (U87). After overnight incubation, cells were treated with only vehicle or indicated MOI of virus or indicated concentration of drugs or reagents. After 2 to 3 days of treatment cell viability was measured by MTT assay (Sigma, Canada) according to the manufacturer's instruction. In brief, cells were incubated with MTT solution for 3 hours at 37oC and then incubated with lysis buffer. After overnight incubation with lysis buffer, cell viability was measured at 595 nm using a plate reader (Envision 2103 Multilabel reader, Perkin Elmer).

### EXAMPLE 16

### U87 XENOGRAFT MODEL.

5 to 6 weeks old female athymic nude mice were obtained from Harlon laboratories. Human glioma U87 cells were implanted subcutaneously into the lower flank. When tumor size reached ~75 to 100 mm3, vehicle or C16 (5 mg/kg) was administered intraperitoneally (IP). 2 days (Figure 14) or 4 days (Figure 15) after initial C16 administration, vehicle or oHSV-1 were injected intratumourally. Tumor volume was then measured using a caliper (Height x Length x Wide/2). At the end of the experiment, mice were euthanized by using CO2 asphyxiation.

### EXAMPLE 17

### TISSUE DNA EXTRACTION & QPCR.

DNA was extracted from 4% paraformaldehyde fixed tumor tissues by using an EZNA tissue DNA kit (Omega Bioteck). Extracted DNAs were subjected to qPCR analysis using Syber green master mix (Invitrogen, Canada) supplemented with ICP27 primers represented by SEQ ID NO:13 (forward) and SEQ ID NO:14 (reverse); and β-actin primers represented by SEQ ID NO:15 (forward) and SEQ ID NO:16 (reverse). Amplification was performed using Quantstudieo 6 Flex qPCR machine (Applied Biosystems, Canada).

### EXAMPLE 18

### IMMUNOHISTOCHEMISTRY.

Harvested tumors were subjected to cryostat sectioning after being fixed for 24 hours with 4% paraformaldehyde. Tissues were fixed for 24 hours with 4% paraformaldehyde, followed by 72 hours incubation with 30% sucrose. Tissues were then embedded in OCT (Sakura tissue tek), sectioned (20 µm) using a cryostat (Leica CM 3050 S), and placed on Fisherbrand^{™} Superfrost^{™} Plus microscope slides (Fisher Scientific, Canada). Slides were then washed with PBS, permeabilized with 0.125% Triton X-100 for 5 minutes and incubated with 5% goat serum (SantaCruz, Canada) for an hour to block unspecific binding. Cells were then incubated overnight with either anti-HSV-1 antibody at 1:50 (Abcam, Cambridge, MA) or anti-f4/80 antibody at 1:50 (Abcam, Cambridge, MA) at 4°C. The following day, after three washing with PBS, sections were incubated with either goat anti-rabbit IgG Alexa Fluor 488 or goat anti-rat IgG Alexa Fluor 568 secondary antibody at 1:500 (Invitrogen, Canada) for an hour at room temperature. After three washing steps, sections were then mounted with Dapi fluromount G (Electron Microscopy Sciences) and visualized and imaged by using a confocal microscope (Olympus, Canada).

### EXAMPLE 19

### STATISTICAL ANALYSIS.

The statistical analyses referenced herein was performed by SPSS 18 or Microsoft Excel and significance (P<0.05) was determined by using independent-samples T test or a significance P<0.001, P<0.01, or P<0.05 was determined using a 2 tailed Student's t-test respectively. Data described in these Examples are expressed as mean ± SD or ± SE.

### EXAMPLE 20

### PRESENCE OF MICROGLIA HINDERS THE ONCOLYTIC EFFICACY OF OHSV-1 AGAINST U87 CELLS.

Efficiency of G207 replication in U87 cells was determined using a one-step viral growth assay (Examples 8 and 9). The results are shown in Figure 14A. G207 anti-proliferative effect was evaluated by MTT assay. A dose dependent anti-proliferative effect was observed and the IC50 was observed for G207 at MOI=1 after 72 hours of infection (Figure 14B). The results confirmed that G207 can effectively replicate in U87 cells, resulting in significant cell lysis. G207 growth in U87 cells in the presence of different numbers of microglia was then measured. Addition of microglia cells in U87 culture inhibited G207 replication in a dose-dependent manner. G207 replication was reduced by 50% and almost 100% by addition of 6.25x103 and 1x105 microglia cells in U87 (5X104) cultures, respectively (Figure 9C). To further confirm that microglia mediated oHSV-1 growth suppression is not strain-specific, different HSV-1 strains were tested, including hRr3 (ICP6 mutated), b17-TK (a TK- mutant) and KOS (wild type). A similar viral replication inhibition among all HSV-1 strains tested was observed (Figure 9D).

### EXAMPLE 21

### MICROGLIA FORM A REPLICATIVE BARRIER TO PREVENT OHSV-1 DISSEMINATION.

Whether oHSV-1 can infect and replicate in microglia was also considered. Both rodent primary cultured microglial cells and BV2 microglia infected with G207 showed LacZ staining, indicating that the virus can enter the cell and express the reporter gene carried by the virus (Figure 10A). Quantification of LacZ in oHSV-1 infected BV2 cells demonstrated that the LacZ expression was dependent on the MOIs of G207 infection (Figure 10B). Concentration dependent HSV-1 infection in microglial cells was also observed by green fluorescent protein (GFP) expressing from a replication deficient HSV-1 as well (data not shown). However, growth assay of G207 in primary cultured rat microglia cells and BV2 cells showed that G207 failed to produce its progeny in microglia (Figure 10C). These results suggest that the virus is internalized by microglia, but their replication is not supported (i.e., that oHSV-1s are capable of internalizing microglias but are unable to replicate therein).

### EXAMPLE 22

### OHSV-1 GENE EXPRESSION PROFILING IN BV2 MICROGLIA CELLS.

To reveal the mechanism by which the HSV-1 replication is prevented in microglial cells, transcript levels of a panel of viral genes in glioma (U87) and microglia (BV2) cells were measured (Example 13). The viral genes included ICP4, ICP27, ICP8, VP5 and Glycoprotein C (gC), representing immediate early, early and late genes, respectively. Quantitative RT-PCR results showed that transcription of ICP27, ICP8, VP5 and gC, but not ICP4, were significantly suppressed in BV2 cells compared to U87 (Figure 11).

### EXAMPLE 23

### C16 OVERCOMES MICROGLIA MEDIATED OHSV-1 REPLICATION BARRIER.

The effects of a PKR inhibitor (C16), a NFkappaB inhibitor (Bay11), and an iNOS inhibitor (aminoguanidine hydrochloride) were tested on G207 replication in BV2 cells (Example 15). Treatment with 1 and 10 µM of C16 significantly enhanced the replication by 9 times and 8 times, respectively (Figure 12A). However, Bay11 and aminoguanidine hydrochloride had no effect on oHSV-1 replication in BV2 cells (Figure 12A). Furthermore, C16 (but not Bay11 nor aminoguanidine hydrochloride) upregulated expression of ICP4 and ICP27 by 1.8 and 25-fold, respectively (Figure 12B). To verify that C16-mediated viral gene transcriptional augmentation is not due to deletions in ICP34.5 and/or ICP6 in G207, the effect of C16 on wild type (KOS) and ICP6 mutated (HrR3) HSV-1 infected BV2 cells was also examined. C16 treatment also upregulated ICP27 expression in KOS and HrR3 infected BV2 cells (Figure 12C). Finally, whether C16 can overcome this microglia-mediated suppression of viral replication in glioma cells was examined. As shown in Figure 12D, G207 viral replication increased by 33% in the glioma-microglia co-cultures treated with C16.

### EXAMPLE 24

### C16 RESCUE OHSV-1 IN MICROGLIAL CELLS BY INHIBITING STAT 1 AND STAT 3 ACTIVATION.

As shown in Figure 13, C16 was also demonstrated to significantly suppress the upregulation of pSTAT1 (Tyr701) and pSTAT3 (Tyr705) induced by oHSV-1 in the BV2 microglia cells, independent of elF2α phosphorylation status. Overall, expression level of STAT 1, but not STAT 3, was upregulated in G2O7 infected microglial cells and that was also reduced after C16 treatment. C16 was also shown to cause inhibition in STAT 1/3 phosphorylation, as confirmed in LPS treated BV2 cells (Figure 16).

### EXAMPLE 25

### C16 SELECTIVELY FACILITATES OHSV-1 REPLICATION IN GLIOMA XENOGRAFT BY OVERCOMING BARRIERS OF TUMOR ASSOCIATED MACROPHAGE.

To demonstrate that the effect of C16 can be translated into enhanced efficacy of intratumoural replication of oHSV in vivo, C16 was injected into animals bearing subcutaneously implanted U87 tumors that received oHSV-1 intratumorally (Example 16). Administration of C16 significantly enhanced oHSV-1 titer in the tumor mass, measured by viral DNA copy number using qPCR (Figure 14A). Immunohistochemistry analysis (Example 18) demonstrated an increased number of replicable HSV-1 cells in animals co-treated with oHSV-1 and 5 mg/kg C16, compared to those treated with oHSV-1 alone. Furthermore, numerous cells with co-localization of HSV-1 and macrophage markers were observed in animals co-treated with oHSV-1 and C16 but not oHSV-1 alone, indicating increased viral replication in the macrophages after C16 treatment (Figure 14B).

### EXAMPLE 26

### C16 SIGNIFICANTLY IMPROVE HUMAN GLIOMA XENOGRAFT REGRESSION.

Finally, whether C16-mediated enhanced oHSV-1 load in tumor is capable of augmenting anti-tumor oncolysis was examined. As shown in Figure 15A, the C16 (5 mg/kg) and oHSV-1 combination treatment group's tumor size was 9.3 fold, 8.2 fold and 6 fold reduced compared to treatment with vehicle, C16 alone, and oHSV-1 alone, respectively, at 50 days post-tumor implantation. Furthermore, qPCR viral titer (Example 17) in different organs of the C16 and oHSV-1 combination treated animals demonstrated that viruses were restricted only in the tumors without spreading to the liver, brain and gastrointestinal track (Figure 15B). Kaplan Meier analysis also revealed the increased survival of the C16 and oHSV-1 combination treatment (Figure 15C).

### EXAMPLE 27

### STAT INHIBITION BY THE COMBINATION OF NF AND OHSV-1

NF is a known inhibitor of STAT1 and STAT3. To evaluate the underlying molecular mechanism of the anti-tumour effect of the NF and oHSV-1 combination, the STAT1 and STAT3 status in U87 cells (Figure 19A) and CT26 cells (Figure 19B) cells was determined after an overnight treatment with NF and oncolytic viruses.

NF caused a dose dependent inhibition of phosphorylation of STAT1/3. NF also effectively suppressed HrR3 induced upregulation of STAT1/3 phosphorylation in both U87 (Figure 19A) and CT26 (Figure 19B) cells. Surprisingly, despite the fact that HrR3 infection elevated STAT1/3 phosphorylation in U87 cells, a combination of high doses of NF and HrR3 (NF 50µM and HrR3 6.25 MOI) reduced STAT1/3 phosphorylation to a level even below that seen with NF alone (Figure 19A). However, HSV-1 infection mediated STAT1/3 phosphorylation augmentation was found time dependent, as STAT1/3 phosphorylation is gradually reduced by time (data not shown).

To further confirm that NF is able to inhibit STAT activation *in vivo,* subcutaneously implanted U87 tumours were treated with either HrR3 alone or in combination with 50 mg/kg NF or 100 mg/kg NF. Expression levels of phosphorylated STAT1/3 in harvested tumour tissues were measured by western blotting. Although pSTAT1 was not detected in tumor tissue, it was evident that STAT3 phosphorylation was increased in the tumor mass treated with HrR3 alone. Again, the virally induced upregulation of pSTAT3 was inhibited in a dose-dependent fashion by the combination of the virus with i.p. injected NF (Figure 19C).

### EXAMPLE 28

### ANTI-TUMOR EFFECT OF OHSV-1 AND NF IN VIVO

CT26 (colon cancer) tumour bearing BALB/C mice were treated with a single dose of HrR3 (oHSV-1) virus (2X10^7 PFU) or vehicle and given daily peritoneal injections of 50mg/kg NF alone or in combination with HrR3 virus (5 mice in each group).Tumor size was measured using calipers (length X height X width /2). Data are the means ± S.E. and statistically significance differences between treatment with the NF and oHSV-1 combination, NF alone, and oHSV-1 alone are indicated by the p value, * P<0.05.

. Increased tumour regression was seen with the combination compared to treatment with NF or HrR3 alone. At day 23 after tumour implantation, 2.2-fold and 1.9-fold greater tumour regression was seen in the NF plus oHSV-1 treated mice compared to mice treated with oHSV-1 alone or NF alone, respectively (Figure 20).

### EXAMPLE 29

### SAFETY OF OHSV-1 AND NF IN VIVO

The safety of combination of NF and oHSV-1 was evaluated. Total DNA was prepared from the tumor, brain, liver and gastrointestinal tract (GT) and subjected to qPCR analysis to measure the amount of viral DNA.

As expected, similarly high levels of HSV-1 DNA were detected in the HrR3 treated tumors with or without NF. While levels of the viral DNA were almost undetectable in the liver and GT with or without NF, a remarkable level was detected in the brains of animals treated with HrR3 alone, and the level was nearly 10-fold less when the virus was combined with NF (Figure 21A). Among 5 mice treated with HrR3 (2X10^7 PFU) alone, one mouse was euthanized due to virus related toxicity. No toxicity was observed in mice treated with the NF and HrR3 combination. Furthermore, when two mice were injected intratumorally with a high dose of HrR3 (1X10^8 PFU), both mice showed signs of severe HSV-1 toxicity (paralysis of the hind leg or immobility) and were terminated immediately. On the other hand, HSV-1 had no toxic effect on mice treated with HrR3 at the same high dose when given in combination with NF (data not shown).

To further confirm that safety is not compromised by NF in the combination, body weight was measured for animals in various course of the treatment. Net body weight was not significantly different in mice treated with the NF and HrR3 combination compared to vehicle or single agent treatment (Figure 21B). These data indicate that NF in combination with HrR3 did not compromise the safety, and even reduced HSV-1 toxicity in the brain.

From the foregoing it will be appreciated that, although specific embodiments have been described herein for purposes of illustration, various modifications may be made without deviating from the spirit and scope of the invention. Accordingly, the invention is not limited except as by the appended claims.

All of the U.S. patents, U.S. patent application publications, U.S. patent applications, foreign patents, foreign patent applications and non-patent publications referred to in this specification are incorporated herein by reference, in their entirety. Such documents may be incorporated by reference for the purpose of describing and disclosing, for example, materials and methodologies described in the publications, which might be used in connection with the presently described invention. The publications discussed above and throughout the text are provided solely for their disclosure prior to the filing date of the present application. Nothing herein is to be construed as an admission that the inventors are not entitled to antedate any referenced publication by virtue of prior invention.

In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

### REFERENCES

1. Yoon SS, Nakamura H, Carrol NM, Bode BP, Chiocca EA and Tanabe KK. An oncolytic herpes simplex virus type 1 selectively destroys diffuse liver metastases from colon carcinoma. The FASEB Journal. 2000; 14(2):301-311.
2. Mineta T, Rabkin SD, Yazaki T, Hunter WD and Martuza RL. Attenuated multi-mutated herpes simplex virus-1 for the treatment of malignant gliomas. Nature medicine. 1995; 1(9):938-943.
3. Kooby DA, Carew JF, Halterman MW, Mack JE, Bertino JR, Blumgart LH, Federoff HJ and Fong Y. Oncolytic viral therapy for human colorectal cancer and liver metastases using a multi-mutated herpes simplex virus type-1 (G207). The FASEB journal. 1999; 13(11):1325-1334.
4. Todo T, Martuza RL, Rabkin SD and Johnson PA. Oncolytic herpes simplex virus vector with enhanced MHC class I presentation and tumor cell killing. Proceedings of the National Academy of Sciences. 2001; 98(11):6396-6401.
5. Mace A, Ganly I, Soutar DS and Brown SM. Potential for efficacy of the oncolytic Herpes simplex virus 1716 in patients with oral squamous cell carcinoma. Head & neck. 2008; 30(8):1045-1051.
6. Harrow S, Papanastassiou V, Harland J, Mabbs R, Petty R, Fraser M, Hadley D, Patterson J, Brown S and Rampling R. HSV1716 injection into the brain adjacent to tumour following surgical resection of high-grade glioma: safety data and long-term survival. Gene therapy. 2004; 11(22): 1648-1658.
7. Nakao A, Kasuya H, Sahin T, Nomura N, Kanzaki A, Misawa M, Shirota T, Yamada S, Fujii T and Sugimoto H. A phase I dose-escalation clinical trial of intraoperative direct intratumoral injection of HF10 oncolytic virus in non-resectable patients with advanced pancreatic cancer. Cancer gene therapy. 2011; 18(3): 167-175.
8. Fong Y, Kim T, Bhargava A, Schwartz L, Brown K, Brody L, Covey A, Karrasch M, Getrajdman G and Mescheder A. A herpes oncolytic virus can be delivered via the vasculature to produce biologic changes in human colorectal cancer. Molecular Therapy. 2009; 17(2):389-394.
9. Andtbacka RH, Kaufman HL, Collichio F, Amatruda T, Senzer N, Chesney J, Delman KA, Spitler LE, Puzanov I and Agarwala SS. Talimogene laherparepvec improves durable response rate in patients with advanced melanoma. Journal of Clinical Oncology. 2015:JCO. 2014.2058. 3377.
10. Gaston DC, Odom Cl, Li L, Markert JM, Roth JC, Cassady KA, Whitley RJ and Parker JN. Production of bioactive soluble interleukin-15 in complex with interleukin-15 receptor alpha from a conditionally-replicating oncolytic HSV-1. PloS one. 2013; 8(11):e81768.
11. Parker JN, Gillespie GY, Love CE, Randall S, Whitley RJ and Markert JM. Engineered herpes simplex virus expressing IL-12 in the treatment of experimental murine brain tumors. Proceedings of the National Academy of Sciences. 2000; 97(5):2208-2213.
12. Passer BJ, Cheema T, Wu S, Wu C, Rabkin SD and Martuza RL. Combination of vinblastine and oncolytic herpes simplex virus vector expressing IL-12 therapy increases antitumor and antiangiogenic effects in prostate cancer models. Cancer gene therapy. 2013; 20(1):17-24.
13. Carew JF, Kooby DA, Halterman MW, Kim S-H, Federoff HJ and Fong Y. A novel approach to cancer therapy using an oncolytic herpes virus to package amplicons containing cytokine genes. Molecular Therapy. 2001; 4(3):250-256.
14. Kambara H, Okano H, Chiocca EA and Saeki Y. An oncolytic HSV-1 mutant expressing ICP34. 5 under control of a nestin promoter increases survival of animals even when symptomatic from a brain tumor. Cancer Research. 2005; 65(7):2832-2839.
15. Fukuhara H, Ino Y, Kuroda T, Martuza RL and Todo T. Triple Gene-Deleted Oncolytic Herpes Simplex Virus Vector Double-Armed with Interleukin 18 and Soluble B7-1 Constructed by Bacterial Artificial Chromosome-Mediated System. Cancer research. 2005; 65(23):10663-10668.
16. Xu R, Yuan Z, Guan Z, Cao Y, Wang H, Hu X, Feng J, Zhang Y, Li F and Chen Z. [Phase II clinical study of intratumoral H101, an E1B deleted adenovirus, in combination with chemotherapy in patients with cancer]. Ai zheng= Aizheng= Chinese journal of cancer. 2003; 22(12):1307-1310.
17. Nemunaitis J, Cunningham C, Buchanan A, Blackburn A, Edelman G, Maples P, Netto G, Tong A, Randlev B and Olson S. Intravenous infusion of a replication-selective adenovirus (ONYX-015) in cancer patients: safety, feasibility and biological activity. Gene therapy. 2001; 8(10):746-759.
18. DeWeese TL, van der Poel H, Li S, Mikhak B, Drew R, Goemann M, Hamper U, DeJong R, Detorie N and Rodriguez R. A phase I trial of CV706, a replication-competent, PSA selective oncolytic adenovirus, for the treatment of locally recurrent prostate cancer following radiation therapy. Cancer research. 2001; 61(20):7464-7472.
19. Ramesh N, Ge Y, Ennist DL, Zhu M, Mina M, Ganesh S, Reddy PS and Yu D-C. CG0070, a conditionally replicating granulocyte macrophage colony-stimulating factor-armed oncolytic adenovirus for the treatment of bladder cancer. Clinical cancer research. 2006; 12(1):305-313.
20. Nemunaitis J, Tong AW, Nemunaitis M, Senzer N, Phadke AP, Bedell C, Adams N, Zhang Y-A, Maples PB and Chen S. A phase I study of telomerase-specific replication competent oncolytic adenovirus (telomelysin) for various solid tumors. Molecular Therapy. 2010; 18(2):429-434.
21. Freytag SO, Khil M, Stricker H, Peabody J, Menon M, DePeralta-Venturina M, Nafziger D, Pegg J, Paielli D and Brown S. Phase I study of replication-competent adenovirus-mediated double suicide gene therapy for the treatment of locally recurrent prostate cancer. Cancer research. 2002; 62(17):4968-4976.
22. Kimball KJ, Preuss MA, Barnes MN, Wang M, Siegal GP, Wan W, Kuo H, Saddekni S, Stockard CR and Grizzle WE. A phase I study of a tropism-modified conditionally replicative adenovirus for recurrent malignant gynecologic diseases. Clinical cancer research. 2010; 16(21):5277-5287.
23. Koski A, Kangasniemi L, Escutenaire S, Pesonen S, Cerullo V, Diaconu I, Nokisalmi P, Raki M, Rajecki M and Guse K. Treatment of cancer patients with a serotype 5/3 chimeric oncolytic adenovirus expressing GMCSF. Molecular Therapy. 2010; 18(10):1874-1884.
24. Lichtenstein D, Spencer J, Doronin K, Patra D, Meyer J, Shashkova E, Kuppuswamy M, Dhar D, Thomas M and Tollefson A. An acute toxicology study with INGN 007, an oncolytic adenovirus vector, in mice and permissive Syrian hamsters; comparisons with wild-type Ad5 and a replication-defective adenovirus vector. Cancer gene therapy. 2009; 16(8):644-654.
25. Kuhn I, Harden P, Bauzon M, Chartier C, Nye J, Thorne S, Reid T, Ni S, Lieber A and Fisher K. Directed evolution generates a novel oncolytic virus for the treatment of colon cancer. PLoS One. 2008; 3(6):e2409.
26. Yu D-C, Chen Y, Seng M, Dilley J and Henderson DR. The addition of adenovirus type 5 region E3 enables calydon virus 787 to eliminate distant prostate tumor xenografts. Cancer research. 1999; 59(17):4200-4203.
27. Li J, Liu H, Zhang X, Xu J, Hu W, Liang M, Chen S, Hu F and Chu D. A phase I trial of intratumoral administration of recombinant oncolytic adenovirus overexpressing HSP70 in advanced solid tumor patients. Gene therapy. 2009; 16(3):376-382.
28. Yuan M, Wong Y, Au G and Shafren D. Combination of intravenously delivered cavatak (coxsackievirus A21) and immune-checkpoint blockade significantly reduces tumor growth and tumor rechallenge. Journal for immunotherapy of cancer. 2015; 3(Suppl 2):P342.
29. Galanis E, Hartmann LC, Cliby WA, Long HJ, Peethambaram PP, Barrette BA, Kaur JS, Haluska PJ, Aderca I and Zollman PJ. Phase I trial of intraperitoneal administration of an oncolytic measles virus strain engineered to express carcinoembryonic antigen for recurrent ovarian cancer. Cancer research. 2010; 70(3):875-882.
30. Myers R, Greiner S, Harvey M, Griesmann G, Kuffel M, Buhrow S, Reid J, Federspiel M, Ames M and Dingli D. Preclinical pharmacology and toxicology of intravenous MV-NIS, an oncolytic measles virus administered with or without cyclophosphamide. Clinical pharmacology and therapeutics. 2007; 82(6):700.
31. Engeland CE, Grossardt C, Veinalde R, Bossow S, Lutz D, Kaufmann JK, Shevchenko I, Umansky V, Nettelbeck DM and Weichert W. CTLA-4 and PD-L1 checkpoint blockade enhances oncolytic measles virus therapy. Molecular Therapy. 2014; 22(11):1949-1959.
32. Grote D, Cattaneo R and Fielding AK. Neutrophils Contribute to the Measles Virus-induced Antitumor Effect Enhancement by Granulocyte Macrophage Colony-stimulating Factor Expression. Cancer research. 2003; 63(19):6463-6468.
33. Freeman Al, Zakay-Rones Z, Gomori JM, Linetsky E, Rasooly L, Greenbaum E, Rozenman-Yair S, Panet A, Libson E and Irving CS. Phase I/II trial of intravenous NDV-HUJ oncolytic virus in recurrent glioblastoma multiforme. Molecular Therapy. 2006; 13(1):221-228.
34. Laurie SA, Bell JC, Atkins HL, Roach J, Bamat MK, O'Neil JD, Roberts MS, Groene WS and Lorence RM. A phase 1 clinical study of intravenous administration of PV701, an oncolytic virus, using two-step desensitization. Clinical Cancer Research. 2006; 12(8):2555-2562.
35. Csatary L, Eckhardt S, Bukosza I, Czegledi F, Fenyvesi C, Gergely P, Bodey B and Csatary C. Attenuated veterinary virus vaccine for the treatment of cancer. Cancer detection and prevention. 1992; 17(6):619-627.
36. Vigil A, Park M-S, Martinez O, Chua MA, Xiao S, Cros JF, Martinez-Sobrido L, Woo SL and Garcia-Sastre A. Use of reverse genetics to enhance the oncolytic properties of Newcastle disease virus. Cancer research. 2007; 67(17):8285-8292.
37. Zhao H, Janke M, Fournier P and Schirrmacher V. Recombinant Newcastle disease virus expressing human interleukin-2 serves as a potential candidate for tumor therapy. Virus research. 2008; 136(1):75-80.
38. Geletneky K, Kiprianova I, Ayache A, Koch R, y Calle MH, Deleu L, Sommer C, Thomas N, Rommelaere J and Schlehofer JR. Regression of advanced rat and human gliomas by local or systemic treatment with oncolytic parvovirus H-1 in rat models. Neuro-oncology. 2010; 12(8):804-814.
39. Goetz C and Gromeier M. Preparing an oncolytic poliovirus recombinant for clinical application against glioblastoma multiforme. Cytokine & growth factor reviews. 2010; 21(2):197-203.
40. Forsyth P, Roldán G, George D, Wallace C, Palmer CA, Morris D, Cairncross G, Matthews MV, Markert J and Gillespie Y. A phase I trial of intratumoral administration of reovirus in patients with histologically confirmed recurrent malignant gliomas. Molecular Therapy. 2008; 16(3):627-632.
41. Reddy PS, Burroughs KD, Hales LM, Ganesh S, Jones BH, Idamakanti N, Hay C, Li SS, Skele KL and Vasko A-J. Seneca Valley virus, a systemically deliverable oncolytic picornavirus, and the treatment of neuroendocrine cancers. Journal of the National Cancer Institute. 2007; 99(21):1623-1633.
42. Tai C-K, Wang WJ, Chen TC and Kasahara N. Single-shot, multicycle suicide gene therapy by replication-competent retrovirus vectors achieves long-term survival benefit in experimental glioma. Molecular Therapy. 2005; 12(5):842-851.
43. Park B-H, Hwang T, Liu T-C, Sze DY, Kim J-S, Kwon H-C, Oh SY, Han S-Y, Yoon J-H and Hong S-H. Use of a targeted oncolytic poxvirus, JX-594, in patients with refractory primary or metastatic liver cancer: a phase I trial. The lancet oncology. 2008; 9(6):533-542.
44. Gomella LG, Mastrangelo MJ, McCUE PA, Maguire HC, Mulholland SG and Lattime EC. Phase I study of intravesical vaccinia virus as a vector for gene therapy of bladder cancer. The Journal of urology. 2001; 166(4):1291-1295.
45. Kirn DH, Wang Y, Le Boeuf F, Bell J and Thorne SH. Targeting of interferon-beta to produce a specific, multi-mechanistic oncolytic vaccinia virus. PLoS Med. 2007; 4(12):e353.
46. Chard LS, Maniati E, Wang P, Zhang Z, Gao D, Wang J, Cao F, Ahmed J, El Khouri M and Hughes J. A vaccinia virus armed with interleukin-10 is a promising therapeutic agent for treatment of murine pancreatic cancer. Clinical Cancer Research. 2015; 21(2):405-416.
47. Kim HS, Kim-Schulze S, Kim DW and Kaufman HL. Host lymphodepletion enhances the therapeutic activity of an oncolytic vaccinia virus expressing 4-1BB ligand. Cancer research. 2009; 69(21):8516-8525.
48. Li J, O'Malley M, Sampath P, Kalinski P, Bartlett DL and Thorne SH. Expression of CCL19 from oncolytic vaccinia enhances immunotherapeutic potential while maintaining oncolytic activity. Neoplasia. 2012; 14(12):1115-IN1111.
49. Gil M, Komorowski MP, Seshadri M, Rokita H, McGray AR, Opyrchal M, Odunsi KO and Kozbor D. CXCL12/CXCR4 blockade by oncolytic virotherapy inhibits ovarian cancer growth by decreasing immunosuppression and targeting cancer-initiating cells. The Journal of Immunology. 2014; 193(10):5327-5337.
50. McCart JA, Mehta N, Scollard D, Reilly RM, Carrasquillo JA, Tang N, Deng H, Miller M, Xu H and Libutti SK. Oncolytic vaccinia virus expressing the human somatostatin receptor SSTR2: molecular imaging after systemic delivery using 111Inpentetreotide. Molecular Therapy. 2004; 10(3):553-561.
51. Gentschev I, Müller M, Adelfinger M, Weibel S, Grummt F, Zimmermann M, Bitzer M, Heisig M, Zhang Q and Yong AY. Efficient colonization and therapy of human hepatocellular carcinoma (HCC) using the oncolytic vaccinia virus strain GLV-1h68. PLoS One. 2011; 6(7):e22069.
52. Conrad SJ, El-Aswad M, Kurban E, Jeng D, Tripp BC, Nutting C, Eversole R, Mackenzie C and Essani K. Oncolytic tanapoxvirus expressing FliC causes regression of human colorectal cancer xenografts in nude mice. Journal of Experimental & Clinical Cancer Research. 2015; 34(1):1.
53. Shin EJ, Wanna GB, Choi B, Aguila D, Ebert O, Genden EM and Woo SL. Interleukin-12 expression enhances vesicular stomatitis virus oncolytic therapy in murine squamous cell carcinoma. The Laryngoscope. 2007; 117(2):210-214.
54. Stephenson K, Barra N, Davies E, Ashkar A and Lichty B. Expressing human interleukin-15 from oncolytic vesicular stomatitis virus improves survival in a murine metastatic colon adenocarcinoma model through the enhancement of anti-tumor immunity. Cancer gene therapy. 2012; 19(4):238-246.
55. Bergman I, Griffin JA, Gao Y and Whitaker-Dowling P. Treatment of implanted mammary tumors with recombinant vesicular stomatitis virus targeted to Her2/neu. International journal of cancer. 2007; 121(2):425-430.
56. Wennier ST, Liu J, Li S, Rahman MM, Mona M and McFadden G. Myxoma virus sensitizes cancer cells to gemcitabine and is an effective oncolytic virotherapeutic in models of disseminated pancreatic cancer. Molecular Therapy. 2012; 20(4):759-768.
57. Tosic V, Thomas DL, Kranz DM, Liu J, McFadden G, Shisler JL, MacNeill AL and Roy EJ. Myxoma Virus Expressing a Fusion Protein of Interleukin-15 (IL15) and IL15 Receptor Alpha Has Enhanced Antitumor Activity. PloS one. 2014; 9(10):e109801.
58. Van Rikxoort M, Michaelis M, Wolschek M, Muster T, Egorov A, Seipelt J, Doerr HW and Cinatl Jr J. Oncolytic effects of a novel influenza A virus expressing interleukin-15 from the NS reading frame. PLoS One. 2012; 7(5):e36506.
59. Goldstein DJ and Weller SK. Herpes simplex virus type 1-induced ribonucleotide reductase activity is dispensable for virus growth and DNA synthesis: isolation and characterization of an ICP6 lacZ insertion mutant. Journal of virology. 1988; 62(1):196-205.
60. YOON SS, NAKAMURA H, CARROLL NM, BODE BP, CHIOCCA EA and TANABE KK. An oncolytic herpes simplex virus type 1 selectively destroys diffuse liver metastases from colon carcinoma. The FASEB Journal. 2000; 14(2):301-311.
61. Aghi M, Visted T, Depinho R and Chiocca E. Oncolytic herpes virus with defective ICP6 specifically replicates in quiescent cells with homozygous genetic mutations in p16. Oncogene. 2008; 27(30):4249-4254.

The present invention will now be described by way of reference to the following clauses:
1. A method of treating cancer, the method comprising simultaneous or sequential administration of an oncolytic virus and a STAT1/3 inhibitor.
2. The method of clause 1, wherein the STAT1/3 inhibitor is a nitrofuran.
3. The method of clause 2, wherein said nitrofuran is nifuroxazide or a derivative or analog thereof.
4. The method of clause 1, wherein the STAT1/3 inhibitor is C16 or a derivative or analog thereof.
5. A method according to any of clauses 1-4, wherein the cancer is a breast cancer, brain cancer, colon cancer, lung cancer, or prostate cancer.
6. The method of any of clauses 1-5, wherein the oncolytic virus is herpes simplex virus, and optionally, HSV-1.
7. A method according to clause 6, wherein the virus has a defective viral ribonuclease reductase gene, and optionally, an otherwise intact ICP34.5 gene.
8. A method according to either clause 6 or 7, wherein the ICP6 gene is modified such that the ICP6 gene is incapable of expressing a functional ICP6 gene product.
9. A method according to any of clauses 6-7, wherein the oncolytic herpes simplex virus is strain HrR3.
10. A pharmaceutical composition comprising an oncolytic virus, a STAT1/3 inhibitor, and a pharmaceutically acceptable carrier.
11. The pharmaceutical composition of clause 10, wherein the STAT1/3 inhibitor is nitrofuran.
12. The pharmaceutical composition of clause 11, wherein the nitrofuran is nifuroxazide or a derivative or analog thereof.
13. The pharmaceutical composition of clause 10, wherein the STAT1/3 inhibitor is C16 or a derivative or analog thereof.
14. The pharmaceutical composition of any of clauses 10-13, wherein the oncolytic virus is an oncolytic herpes simplex virus.
15. The pharmaceutical composition according to clause 14, wherein the virus has a defective viral ribonuclease reductase gene, and optionally, an otherwise intact ICP34.5 gene.
16. The pharmaceutical composition according to clauses 14 or 15, wherein the ICP6 gene is modified such that the ICP6 gene is incapable of expressing a functional ICP6 gene product.
17. A kit comprising a predetermined amount of an oncolytic virus and a predetermined amount of chemotherapeutic agent, wherein the chemotherapeutic agent is a STAT1/3 inhibitor.
18. The kit according to clause 17, wherein the kit comprises a predetermined amount of oncolytic herpes simplex virus and a predetermined amount of chemotherapeutic agent, wherein the chemotherapeutic agent is a STAT1/3 inhibitor.
19. The kit according to clause 17 or 18, wherein the STAT1/3 inhibitor is a nitrofuran.
20. The pharmaceutical composition of clause 19, wherein the nitrofuran is nifuroxazide or a derivative or analog thereof.
21. The pharmaceutical composition of clause 17, wherein the STAT1/3 inhibitor is C16 or a derivative or analog thereof.
22. A method for improving efficacy of an oncolytic virotherapy, which comprises the steps of:
   (a) administering an oncolytic virus to a subject; and
   (b) administering a STAT1/3 inhibitor in an amount that is effective to reduce microglia- or macrophage-mediated suppression of replication of the oncolytic virus.
23. The method of clause 22, wherein the oncolytic virus and STAT1/3 inhibitor are administered together.
24. The method of clause 22, wherein the oncolytic virus and STAT1/3 inhibitor are administered in series.
25. The method of clause 22, wherein the STAT1/3 inhibitor is C16, salt forms thereof, prodrugs thereof, or derivatives thereof.
26. The method of clause 22, wherein the STAT1/3 inhibitor is nitrofuran.
27. The method of clause 26, wherein the nitrofuran is nifuroxazide or a derivative or analog thereof.

## Claims

1. A oncolytic virus and a STAT1/3 inhibitor for use in a method of treating cancer, comprising simultaneous or sequential administration of the oncolytic virus and a STAT1/3 inhibitor.

2. The method of claim 1, wherein the STAT1/3 inhibitor is a nitrofuran,
particularly wherein said nitrofuran is nifuroxazide or a derivative or analog thereof,
or wherein the STAT1/3 inhibitor is C16 or a derivative or analog thereof.

3. A method according to any of claims 1-2, wherein the cancer is a breast cancer, brain cancer, colon cancer, lung cancer, or prostate cancer.

4. The method of any of claims 1-3, wherein the oncolytic virus is herpes simplex virus, and optionally, HSV-1,
particularly wherein the virus has a defective viral ribonuclease reductase gene, and optionally, an otherwise intact ICP34.5 gene,

5. A method according to claim 4, wherein the ICP6 gene is modified such that the ICP6 gene is incapable of expressing a functional ICP6 gene product,
or wherein the oncolytic herpes simplex virus is strain HrR3.

6. A pharmaceutical composition comprising an oncolytic virus, a STAT1/3 inhibitor, and a pharmaceutically acceptable carrier.

7. The pharmaceutical composition of claim 6, wherein the STAT1/3 inhibitor is nitrofuran,
particularly wherein the nitrofuran is nifuroxazide or a derivative or analog thereof,
or wherein the STAT1/3 inhibitor is C16 or a derivative or analog thereof.

8. The pharmaceutical composition of any of claims 6-7, wherein the oncolytic virus is an oncolytic herpes simplex virus,
particularly wherein the virus has a defective viral ribonuclease reductase gene, and optionally, an otherwise intact ICP34.5 gene,
and/or wherein the ICP6 gene is modified such that the ICP6 gene is incapable of expressing a functional ICP6 gene product.

9. A kit comprising a predetermined amount of an oncolytic virus and a predetermined amount of chemotherapeutic agent, wherein the chemotherapeutic agent is a STAT1/3 inhibitor.

10. The kit according to claim 9, wherein the kit comprises a predetermined amount of oncolytic herpes simplex virus and a predetermined amount of chemotherapeutic agent, wherein the chemotherapeutic agent is a STAT1/3 inhibitor.

11. The kit according to claim 9 or 10, wherein the STAT1/3 inhibitor is a nitrofuran,
particularly wherein the nitrofuran is nifuroxazide or a derivative or analog thereof.

12. The pharmaceutical composition of claim 9, wherein the STAT1/3 inhibitor is C16 or a derivative or analog thereof.

13. A oncolytic virus and STAT1/3 inhibitor for use in a method of improving efficacy of an oncolytic virotherapy, which comprises the steps of:
(a) administering the oncolytic virus to a subject; and
(b) administering the STAT1/3 inhibitor in an amount that is effective to reduce microglia- or macrophage-mediated suppression of replication of the oncolytic virus.

14. The method of claim 13, wherein the oncolytic virus and STAT1/3 inhibitor are administered together,
or wherein the oncolytic virus and STAT1/3 inhibitor are administered in series,
or wherein the STAT1/3 inhibitor is C16, salt forms thereof, prodrugs thereof, or derivatives thereof.

15. The method of claim 13, wherein the STAT1/3 inhibitor is nitrofuran,
particularly wherein the nitrofuran is nifuroxazide or a derivative or analog thereof.
